# EUROPÄISCHE PATENTSCHRIFT

(11) **EP 1 561 450 B1**
(45) Veröffentlichungstag und Bekanntmachung des Hinweises auf die Patenterteilung: **28.09.2011**
(21) Anmeldenummer: 04106847.9
(22) Anmeldetag: 22.12.2004
(51) Int. Cl.: A61K 8/37, A61K 8/39, A61K 8/06, A61K 8/55, A61K 8/81, A61Q 19/10, A61Q 1/14, A61K 8/02

(54) **Dünnflüssige O/W-Emulsion**
Low viscosity O/W- Emulsion
Emulsion huile-dans-eau à faible viscosité

(30) Priorität: 21.01.2004 DE 102004003435
(43) Veröffentlichungstag der Anmeldung: 10.08.2005
(73) Patentinhaber: Beiersdorf AG, 20253 Hamburg (DE)
(72) Erfinder: Kohlhase, Silke, 22523, Hamburg (DE); Schäfer, Andreas, 22299, Hamburg (DE); Hahn, Ingo, 25421, Pinneberg (DE); Storbeck, Celina, 25474, Bönniggstedt (DE); Morgenroth, Susanne, 22848, Norderstedt (DE); Hain, Ulrich, 22045, Hamburg (DE)

(56) Entgegenhaltungen:
- EP-A- 1 449 516
- WO-A-02/066007
- WO-A-03/017952
- WO-A-03/020224
- WO-A-03/030858
- DE-A1- 10 307 468
- DE-A1- 19 949 281

## Beschreibung

Die vorliegende Erfindung betrifft eine dünnflüssige Öl-in-Wasser- (O/W-) Emulsion.

Die Produktion von kosmetischen Reinigungsmitteln zeigt seit Jahren eine steigende Tendenz. Dies ist vor allem auf das zunehmende Gesundheitsbewußtsein und Hygienebedürfnis der Verbraucher zurückzuführen.

Reinigung des menschlichen Körpers bedeutet das Entfernen von (Umwelt-) Schmutz und bewirkt damit eine Erhöhung des psychischen und physischen Wohlbefindens. Die Reinigung der Oberfläche von Haut und Haaren ist ein sehr komplexer, von vielen Parametern abhängiger Vorgang. Zum einen sollen von außen kommende Substanzen wie beispielsweise Kohlenwasserstoffe oder anorganische Pigmente aus unterschiedlichsten Umfeldern sowie Rückstände von Kosmetika oder auch unerwünschte Mikroorganismen möglichst vollständig entfernt werden. Zum anderen sind körpereigene Ausscheidungen wie Schweiß, Sebum, Haut- un d Haarschuppen ohne tief greifende Eingriffe in das physiologische Gleichgewicht der Haut abzuwaschen.

Zur Reinigung der Haut und Hautanhangsgebilde finden neben tensidhaltigen Reinigungszubereitungen Emulsionen in Form von Reinigungsemulsionen Verwendung. Sie werden meist der Gesichtsreinigung und insbesondere zur Entfernung von dekorativer Kosmetik eingesetzt.

Reinigungsemulsionen haben, im Gegensatz zu anderen Reinigungszubereitungen wie beispielsweise Seife, den Vorteil besonders hautverträglich zu sein, da sie in ihrer lipophilen Phase pflegende Öle und unpolare Wirkstoffe wie Vitamin E enthalten können.

Eine besonders weit verbreitete Form kosmetischer Emulsionen stellen dünnflüssige Öl-in-Wasser-Emulsionen (O/W-Emulsionen) dar. Dünnflüssige O/W-Emulsionen basieren in der Regel auf einem Emulgatorsystem aus Ceteareth -20, Ceteareth 12, Cetearylalkohol, Cetylpalmitate und Glycerylstearat. Weitere O/W-Emulgatorsysteme sind: Triceteareth-4 Phosphate; Trilaureth-4 Phosphate; Glyceryl Isostearate + Isoceteth-20; PEG-60 Hydrogenated Castor Oil oder Polysorbate 20.

Derartige Zubereitungen des Standes der Technik haben jedoch den Nachteil, dass sie, nicht zuletzt aufgrund ihres relativ hohen Emulgatorgehaltes, ein klebriges Hautgefühl aufweisen und wenig Pflege bieten. Ferner weisen derartige Zubereitungen in der Regel keine allzu hohe Lagerstabilität (Langzeitstabilität) auf.

Außerdem sind nach dem Stande der Technik hochviskose O/W-Emulsionen mit Polyethylenglykolestern als Emulgatoren bekannt. Emulsionen mit Polyethylenglykolestern als Emulgatoren haben den Vorteil, dass sie langzeitstabil und elektrolytkompatibel sind. Ferner besitzen sie ein reichhaltiges, nicht klebriges Hautgefühl und weisen eine gute Hautverträglichkeit auf.

Derartige Zubereitungen haben jedoch den Nachteil, dass sie aufgrund Ihrer Zähflüssigkeit nur ein beschränktes Anwendungsspektrum abdecken. Sie sind weder versprühbar noch eignen sie sich als Tränkmedium für Tücher.

Um die Probleme des Standes der Technik zu lösen wurden in der Vergangenheit unterschiedliche Lösungswege beschritten. Beispielsweise wurden Pickering-Pigmente als Emulgatoren eingesetzt (DE 102 38 649.8). Ein weiterer Lösungsansatz bestand darin, eine hohe Konzentration an Emulgator (2 Gewichts-% und mehr) in Kombination mit Fettalkoholen einzusetzen (DE 198 02 206 und DE 101 29 973) oder W/O-Emulsionen zu verwenden (DE 101 54 627).

Eine besondere Produktform für Reinigungszubereitungen stellen feste Reinigungssubstrate bzw. -textilien dar, insbesondere Tücher. Diese können bereits vom Hersteller mit der Reinigungszubereitung getränkt sein (eine Kombination, für die im Rahmen der vorliegenden Erfindung auch der Begriff "Reinigungsartikel" verwendet wird) und haben dadurch den Vorteil, dass in ihnen die Zubereitung bereits in der richtigen Dosierung vorgegeben ist. Außerdem vermeiden sie den Nachteil von in Flaschen aufbewahrten Zubereitungen, deren Verpackung zerbrechen und deren Inhalt "auslaufen" kann. Zu den weiteren Vorteilen von Reinigungssubstraten/Textilien zählen auch die Umstände, dass sie sich bequem in abgezählter Menge mit auf Reisen nehmen lassen und für ihre Anwendung in der Regel kein Wasser mehr erforderlich ist. Reinigungssubstrate/Tücher werden aus Textilien hergestellt. Textilien können gewebt, gestrickt oder gewirkt sein oder als Verbundstoff (engl. nonwoven textile) vorliegen. Meist werden (aus Kostengründen) Verbundstoffe verwendet. Bei Verbundstoffen erfolgt die Gewebebildung nicht durch Kette und Schuss oder Maschenbildung, sondern durch Verschlingung, und/oder kohäsive und/oder adhäsive Verbindung von Textilfasern. Verbundstoffe können nach der DIN 61210 T2 in Vlies, Papier Watte und Filz unterschieden werden. Vliese sind lockere Materialien aus Spinnfasern (d.h. Faser mit begrenzter Länge) oder Filamenten (Endlosfasern), meist aus Polypropylen, Polyester oder Viskose hergestellt, deren Zusammenhalt im allgemeinen durch die den Fasern eigene Haftung gegeben ist. Hierbei können die Einzelfasern eine Vorzugsrichtung aufweisen (orientierte oder Kreuzlage-Vliese) oder ungerichtet (Wirrvliese) sein. Die Vliese können mechanisch verfestigt werden durch Vernadeln, Vermaschen oder durch Verwirbeln mittels scharfer Wasserstrahlen. Adhäsiv verfestigte Vliese entstehen durch Verkleben der Fasern mit flüssigen Bindemitteln (z.B. Acrylat-Polymere, SBR/NBR, Polyvinylester, Polyurethan-Dispersionen) oder durch Schmelzen oder Auflösen von sogenannten Bindefasern, die dem Vlies bei der Herstellung beigemischt wurden. Bei der kohäsiven Verfestigung werden die Faseroberflächen durch geeignete Chemikalien angelöst und durch Druck verbunden oder bei erhöhter Temperatur verschweißt [J. Falbe, M. Regnitz: Römpp-ChemieLexikon, 9. Aufl. Thieme-Verlag, Stuttgart (1992)].

Mit kosmetischen Zubereitungen imprägnierte Substrate und insbesondere Tücher können auf unterschiedlichen Wegen hergestellt werden: Im so genannten "Tauch-Verfahren" wird das Tuch in einem Tauchbad eingetaucht oder durch ein Bad gezogen. Dieses Verfahren eignet sich insbesondere für Papiertücher und weniger für Vliese, da letztere zu viel Flüssigkeit (=Zubereitung) aufnehmen und sich in Umverpackung anschließend Pfützen von wieder freigesetzter Zubereitung finden.

Eine zweite Variante stellt das "Sprüh-Verfahren" dar, bei dem die Zubereitung auf das vorbeilaufende Tuch aufgesprüht wird. Diese Verfahren eignet sich für alle Textilien, doch können keine stark schäumenden Zubereitungen auf das Tuch aufgebracht werden, da die Schaumentwicklung beim Sprühverfahren zu groß wird.

Als weitere Methode kommen so genannte Abstreifmethoden zum Einsatz. Dort laufen Vlies oder Tuchbahnen an Abstreifblechen, -balken oder -düsen vorbei, die kontinuierlich mit Imprägnierungslösung beladen werden. Unterschiedliche Imprägnierungsgrade lassen sich u. a. durch Variation des Anpressdruckes und der Tuchzuggeschwindigkeit einstellen.

Nach dem Stande der Technik hergestellte Substrate/Tücher, die mit Reinigungszubereitungen oder anderen kosmetischen Zubereitungen imprägniert sind (= Reinigungsartikel), haben jedoch eine Reihe von Nachteilen: So sind mit hergebrachten Tränkungsmedien behandelte Substrate/Tücher durch eine wässrig/leere Sensorik gekennzeichnet. Sie weisen eine nur unbefriedigende Reinigungsleistung, insbesondere für wasserfeste Kosmetikprodukte (z.B. Mascaras, etc.) auf. Darüber hinaus sind derartige Produkte durch eine nur eingeschränkte Lagerstabilität gekennzeichnet, die sich insbesondere durch Ausbluten (Draining) der Produkte bemerkbar macht. Dies führt zu ungleichmässig getränkten Produkten, die vom Konsumenten als qualitativ minderwertig empfunden werden. Ferner sind herkömmliche Tuchprodukte nur schwierig konsumentengerecht und langzeitstabil zu konservieren.

Es war deshalb die Aufgabe der vorliegenden Erfindung, die Nachteile des Standes der Technik zu beseitigen. Insbesondere war es die Aufgabe, dünnflüssige O/W-Reinigungsemulsionen sowie Reinigungstextilien zu entwickeln, welche die Mängel des Standes der Technik vermeiden. Ferner war es die Aufgabe der vorliegenden Erfindung, dünnflüssige, besonders lagerstabile O/W-Emulsionen zu entwickeln, die sich durch ein allenfalls minimal klebriges Hautgefühl aufweisen und besonders hautverträglich sind.

Nicht zuletzt wurde die Anforderung an die O/W-Emulsion gestellt, sowohl als Tränkzubereitung für Tücher und andere Textilien geeignet zu sein, als auch mittels einer Sprühvorrichtung (z.B. Aerosoldose, Pumpspray) versprühbar zu sein.

Überraschend gelöst werden die Aufgaben durch eine kosmetische und/oder dermatologische Öl-in-Wasser- (O/W-) Emulsion mit einer Viskosität von 10 bis 1500 mPaS, enthaltend
a) einen oder mehrere Emulgatoren gewählt aus der Gruppe Glycerylstearatcitrat, Polyglycerinmethylglucosedistearat, Triceteareth-4-phosphat,
b) einem Homo- oder Copolymer der Arcrylsäure und/oder ihrer Derivate als polymeren Stabilisator,
wobei die Gesamtkonzentration der Summe aus Emulgator a) und polymeren Stabilisator b) zwischen 0,01 und 1,5 Gewichts-%, bezogen auf das Gesamtgewicht der Emulsion beträgt und die Emulsion frei ist von Fettalkoholen mit einer Kettenlänge von 12 bis 20 Kohlenstoffatomen

Zwar kennt der Fachmann die WO 02/066007, WO 03/017952, EP 1449516, DE 19949281, DE 10307468, WO 03/030858, WO 03/020224, doch konnten diese Schriften nicht den Weg zur vorliegenden Erfindung weisen.

Die im Rahmen der vorliegenden Schrift aufgeführten Viskositätswerte der Zubereitungen und Einzelsubstanzen wurden mit Hilfe eines Viskosimeters des Typs Viskotester VT 02 der Gesellschaft Haake ermittelt (Temperatur: 25°C, Spindeldurchmesser 23 mm, Rotorgeschwindigkeit 62.5 1/min).

Es ist erfindungsgemäß vorteilhaft, wenn als Emulgator a) Glycerylstearatcitrat eingesetzt wird. Glycerinstearatcitrat (CAS 39175-72-9, INCI: Glyceryl Stearate Citrate) kann u.a. unter dem Handelsnamen Imwitor 370 bei der Firma Hüls erworben werden. Erfindungsgemäß vorteilhaft wird Glycerylstearatcitrat in einer Konzentration von 0,01 bis 1,5 Gewichts-% und erfindungsgemäß bevorzugt in einer Konzentration von 0,1 bis 1,3 Gewichts-%, jeweils bezogen auf das Gesamtgewicht der Zubereitung, eingesetzt.

In einer weiteren erfindungsgemäß vorteilhaften Ausführungsform der vorliegenden Erfindung wird als Emulgator a) Polyglycerinmethylglucosedistearat eingesetzt. Polyglycerinmethylglucosedistearat (INCI: Polyglycerol Methyl Glucose Distearate) kann u.a. unter der Handelsbezeichnung Tego Care-450 bei der Firma Goldschmidt erworben werden. Erfindungsgemäß vorteilhaft wird Polyglycerinmethylglucosedistearat in einer Konzentration von 0,01 bis 1,5 Gewichts-% und erfindungsgemäß bevorzugt in einer Konzentration von 0,1 bis 1,3 Gewichts-%, jeweils bezogen auf das Gesamtgewicht der Zubereitung, eingesetzt.

In einer weiteren erfindungsgemäß vorteilhaften Ausführungsform der vorliegenden Erfindung wird als Emulgator a) Triceteareth-4-phosphat eingesetzt. Triceteareth -4-phosphat (INCI: Triceteareth-4 phosphate) wird z.B. von der Firma Clariant unter dem Handelsnamen Hostaphat KW 340D im Handel angeboten. Es ist erfindungsgemäß vorteilhaft Triceteareth-4-phosphat in einer Konzentration von 0,01 bis 1,5 Gewichts-% und erfindungsgemäß bevorzugt in einer Konzentration von 0,1 bis 1,3 Gewichts-%, jeweils bezogen auf das Gesamtgewicht der Zubereitung, einzusetzen.

In den erfindungsgemäßen Emulsionen können polymere Stabilisatoren eingesetzt werden. Erfindungsgemäß vorteilhaft sind z.B. Homo- oder Copolymer der Arcrylsäure.

Beispielhaft zu nennen sind hier die aus der Gruppe der sogenannten Carbomere oder Carbopole (Carbopol® ist eigentlich eine eingetragene Marke der

B. F. Goodrich Company) stammenden Verbindungen. Die erfindungsgemäß vorteilhaften Acrylat-Alkylacrylat-Copolymere zeichnen sich durch die folgende Struktur aus:

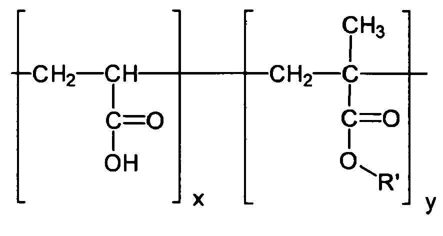

Darin stellen R' einen langkettigen Alkylrest und x und y Zahlen dar, welche den jeweiligen stöchiometrischen Anteil der jeweiligen Comonomere symbolisieren.

Erfindungsgemäß bevorzugt sind Acrylat-Copolymere und/oder Acrylat-Alkylacrylat-Copolymere, welche unter den Handelbezeichnungen Carbopol® 1382, Carbopol® 981 und Carbopol® 5984 von der B. F.Goodrich Company erhältlich sind, sowie Polyacrylate aus der Gruppe der Carbopole der Typen 980, 981, 1382, 2984, 5984 sowie Carbomer 2001. Weiterhin vorteilhaft sind Sodium Polyacrylate erhältlich z.B. unter der Handelsbezeichnung Cosmedia SP von Cognis.

Vorteilhaft sind ferner Copolymere/Crosspolymere umfassend Sodium Acrylate wie Aqua SF-1 von Noveon

Ferner sind bevorzugt Copolymere aus C₁₀-₃₀-Alkylacrylaten und einem oder mehreren Monomeren der Acrylsäure, der Methacrylsäure oder deren Ester, die kreuzvernetzt sind mit einem Allylether der Saccharose oder einem Allylether des Pentaerythrit.

Besonders Vorteilhaft sind Verbindungen, die die INCI-Bezeichnung "Acrylates/C₁₀₋₃₀ Alkyl Acrylate Crosspolymer" tragen. Insbesondere vorteilhaft sind die unter den Handelsbezeichnungen Pemulen TR1 und Pemulen TR2, Carbopol 1328, Ultrez 21 bei der B. F. Goodrich Company sowie unter der Handelsbezeichnung Tegocarbomer TC 341 ER von Goldschmidt / Degussa erhältlichen.

Weiterhin sind vorteilhaft AMPS-Polymere und Copolymere (AMPS : Acrylamidomethylpropylsulfonsäure) wie z.B. das unten den den Chemical Abstracts Registraturnummern 58374-69-9, 13162-05-5 und 88-12-0 abgelegte und unter der

Handelsbezeichnung Aristoflex® AVC verfügbare Polymer der Gesellschaft Clariant GmbH (INCI :Ammoniumacryloyldimethyltaurate/Vinylpyrrolidoncopolymere)

Das erfindungsgemäß vorteilhafte AmmoniumacryloyldimethyltaurateNinylpyrrolidoncopolymer weist die Summenformel [C₇H₁₆N₂SO₄]ₙ [C₆H₉NO]ₘ auf, einer statistischen Struktur wie folgt entsprechend :

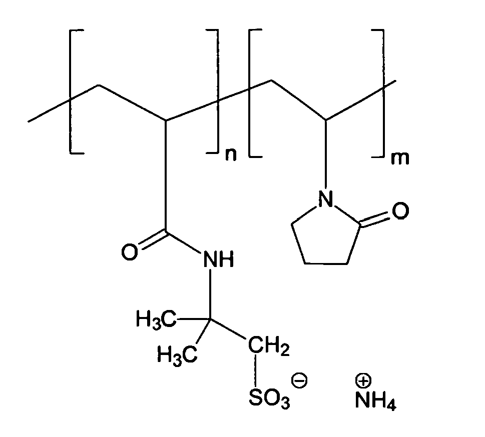

Vorteilhaft sind ferner Copolymere/Crosspolymere umfassend Acryloyldimethyl Taurate, wie beispielsweise Simugel ® EG oder Simugel ® EG von der Gesellschaft Seppic S.A.

Die zuvor erwähnten synthetische Polyelektrolyte werden in der Regel, wenn nicht bereits vorneutralisiert, durch Zugabe von geeigneten anorganischen oder organischen Basen, wie z.B. Natronlauge, Kalilauge, Triethylamin oder Ammoniakwaser zumindest teilneutralisiert und somit in die im Sinne der Anwendung vorteilhafte ionische Salzform übergeführt.

Es ist erfindungsgemäß vorteilhaft, wenn die erfindungsgemäße Emulsion dadurch gekennzeichnet ist, dass die Konzentration an polymeren Stabilisator 0,001 bis 1,5 Gewichts-% und bevorzugt von 0,05 Bis 0,2 Gewichts-%, jeweils bezogen auf das Gesamtgewicht der Zubereitung beträgt.

Erfindungsgemäß vorteilhaft beträgt das Gewichtsverhältnis von Emulgator a) zu polymeren Stabilisator b) von 20:1 bis 1:1.

Die erfindungsgemäßen Emulsionen können erfindungsgemäß vorteilhaft weitere Bestandteile enthalten:

Die erfindungsgemäßen Emulsionen können als wässrige Phase neben Wasser erfindungsgemäß vorteilhaft auch andere Inhaltsstoffe enthalten, beispielsweise Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol und Glycerin.

Die erfindungsgemäße Emulsion enthält ein oder mehrere Öl-, Lipid-, und/oder Wachskomponenten gleicher oder unterschiedlicher Polarität. Polare Öle, sind beispielsweise solche aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z.B. Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Besonders vorteilhafte polare Lipide im Sinne der vorliegenden Erfindung sind alle nativen Lipide, wie z. B. Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl, Maiskeimöl, Avocadoöl und dergleichen sowie die im folgenden aufgelisteten.

| **Hersteller** | **Handelsname** | **INCI-Name** | **Polarit ät [mN/m]** |
|---|---|---|---|
| Condea Chemie | Isofol 14 T | Butyl Decanol (+) Hexyl Octanol (+) | 19,8 |
| | | Hexyl Decanol (+) Butyl Octanol | |
| Lipochemicals | Lipovol MOS-130 | Tridecyl Stearate(+) Tridecyl | 19,4 |
| INC. / USA | | Trimellitate(+) Dipentaerythrityl | |
| (Induchem) | | Hexacaprylate/Hexacaprate | |
| | Ricinusoel | | 19,2 |
| CONDEA Chemie | Isofol Ester 0604 | | 19,1 |
| Huels | Miglyol 840 | Propylene Glycol | 18,7 |
| CONDEA Chemie | | Dicaprylate/Dicaprate | |
| *CONDEA* | Isofol 12 | Butyl Octanol | 17,4 |
| *Chemie* | | | |
| Goldschmidt | Tegosoft SH | Stearyl Heptanoate | 17,8 |
| | Avocadooel | | 14,5 |
| Henkel Cognis | Cetiol B | Dibutyl Adipate | 14,3 |
| ALZO (ROVI) | Dermol 488 | PEG 2 Diethylenhexanoate | 10,1 |
| Condea Augusta | Cosmacol ELI | C12-13 Alkyl Lactate | 8,8 |
| S.P.A. | | | |
| ALZO (ROVI) | Dermol 489 | Diethylen Glycol Dioctanoate(/ | 8,6 |
| | | Diisononanoate | |
| Condea Augusta | Cosmacol ETI | Di-C12/13 Alkyl Tartrate | 7,1 |
| S.P.A. | | | |
| Henkel Cognis | Emerest 2384 | Propylene Glycol Monoisostearate | 6,2 |
| Henkel Cognis | Myritol 331 | Cocoglycerides | 5,1 |
| Unichema | Prisorine 2041 | Triisostearin | 2,4 |
| | GTIS | | |

Ferner kann die Ölphase vorteilhaft gewählt werden aus der Gruppe der Dialkylether. Es ist insbesondere vorteilhaft, wenn die Ölphase einen Gehalt an C₁₂₋₁₅-Alkylbenzoat aufweist oder vollständig aus diesem besteht.

Ferner kann die Ölphase vorteilhaft gewählt werden aus der Gruppe der Guerbetalkohole. Guerbetalkohole sind benannt nach Marcel Guerbet, der ihre Herstellung erstmalig beschrieb. Sie entstehen nach der Reaktionsgleichung 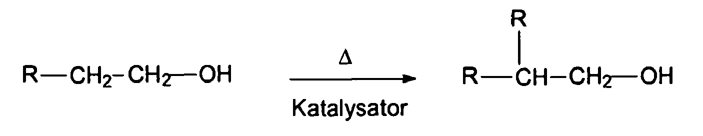 durch Oxidation eines Alkohols zu einem Aldehyd, durch Aldol-Kondensation des Aldehyds, Abspaltung von Wasser aus dem Aldol- und Hydrierung des Allylaldehyds. Guerbetalkohole sind selbst bei niederen Temperaturen flüssig und bewirken praktisch keine Hautreizungen. Vorteilhaft können sie als fettende, überfettende und auch rückfettend wirkende Bestandteile in Haut- und Haarpflegemitteln eingesetzt werden.

Die Verwendung von Guerbet-Alkoholen in Kosmetika ist an sich bekannt. Solche Species zeichnen sich dann meistens durch die Struktur 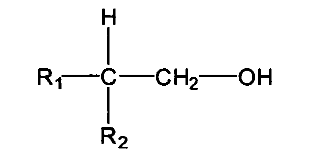 aus. Dabei bedeuten R₁ und R₂ in der Regel unverzweigte Alkylreste.

Erfindungsgemäß vorteilhaft werden der oder die Guerbet-Alkohole gewählt aus der Gruppe, bei denen
R₁ = Propyl, Butyl, Pentyl, Hexyl, Heptyl oder Octyl und
R₂ = Hexyl, Heptyl, Octyl, Nonyl, Decyl, Undecyl, Dodecyl, Tridecyl oder Tetradecyl.

Erfindungsgemäß bevorzugte Guerbet-Alkohole sind das 2-Butyloctanol - es hat die chemische Struktur 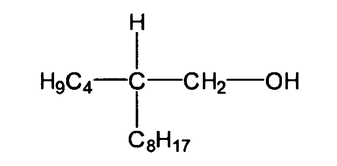 und ist beispielsweise unter der Handelsbezeichnung Isofol^{®} 12 von der Gesellschaft Condea Chemie GmbH erhältlich - und das 2-Hexyldecanol - es hat die chemische Struktur 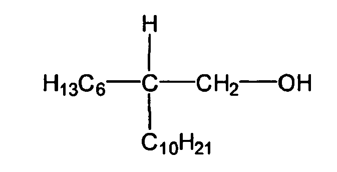 und ist beispielsweise unter der Handelsbezeichnung Isofol® 16 von der Gesellschaft Condea Chemie GmbH erhältlich. Auch Mischungen von erfindungsgemäßen Guerbet-Alkoholen sind erfindungsgemäß vorteilhaft zu verwenden. Mischungen aus 2-Butyloctanol und 2-Hexyldecanol sind beispielsweise unter der Handelsbezeichnung Isofol® 14 von der Gesellschaft Condea Chemie GmbH erhältlich.

Die Gesamtmenge an Guerbet-Alkoholen in den fertigen kosmetischen oder dermatologischen Zubereitungen wird vorteilhaft aus dem Bereich bis 25,0 Gew.-%, bevorzug 0,5 - 15,0 Gew.-% gewählt, bezogen auf das Gesamtgewicht der Zubereitungen.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Besonders vorteilhafte mittelpolare Lipide im Sinne der vorliegenden Erfindung sind die im folgenden aufgelisteten Substanzen:

| **Hersteller** | **Handelsname** | **INCI-Name** | **Polarit ät [mN/m]** |
|---|---|---|---|
| Stearinerie Dubois | DUB VCI 10 | Isodecyl Neopentanoate | 29,9 |
| Fils | | | |
| ALZO (ROVI) | Dermol IHD | Isohexyldecanoate | 29,7 |
| ALZO (ROVI) | Dermol 108 | Isodecyl Octanoate | 29,6 |
| | Dihexyl Ether | Dihexyl Ether | 29,2 |
| ALZO (ROVI) | Dermol 109 | Isodecyl 3,5,5 Trimethyl Hexanoate | 29,1 |
| Henkel Cognis | Cetiol SN | Cetearyl Isononanoate | 28,6 |
| Unichema | Isopropylpalmitat | Isopropylpalmitat | 28,8 |
| Dow Coming | DC Fluid 345 | Cyclomethicone | 28,5 |
| Dow Coming | Dow Coming Fluid | Cyclopolydimethylsiloxan | 28,5 |
| | 244 | | |
| Nikko Chemicals | Jojobaöl Gold | | 26,2 |
| Superior Jojoba | | | |
| Oil Gold | | | |
| Wacker | Wacker AK 100 | Dimethicone | 26,9 |
| ALZO (ROVI) | Dermol 98 | 2- Ethylhexanosäure 3,5,5 | 26,2 |
| | | Trimethylester | |
| Dow Coming | Dow Coming Fluid 246 | Offen | 25,3 |

| | | | |
|---|---|---|---|
| ALZO (ROVI) | Dermol 139 | Isotridecyl 3,5,5 | 24,5 |
| | | Trimethylhexanonanoate | |
| | Cegesoft C24 | Octyl Palmitate | 23,1 |
| Gattefossé | M.O.D. | Octyldodeceyl Myristate | 22,1 |
| | Macadamia Nut Oil | | 22,1 |
| Bayer AG, | *Silikonöl VP 1120* | Phenyl Trimethicone | 22,7 |
| Dow Coming | | | |
| CONDEA Chemie | Isocarb 12 | Butyl Octanoicacid | 22,1 |
| Henkel Cognis | Isopropylstearat | Isopropyl Stearate | 21,9 |
| WITCO, | Finsolv TN | C12-15 Alkyl Benzoate | 21,8 |
| Goldschmidt | | | |
| Dr. Straetmans | Dermofeel BGC | Butylene Glycol Caprylate/Caprate | 21,5 |
| Unichema | Miglyol 812 | Caprylic/Capric Triglyceride | 21,3 |
| Huels | | | |
| Trivent (über S. | Trivent OCG | Tricaprylin | 20,2 |
| Black) | | | |
| ALZO (ROVI) | Dermol 866 | PEG " Diethylhexanoate/ | 20,1 |
| | | Diisononanoate/ Ethylhexyl | |
| | | Isononanoate | |

Unpolare Öle sind beispielsweise solche, welche gewählt werden aus der Gruppe der verzweigten und unverzweigten Kohlenwasserstoffe und -wachse, insbesondere Vaseline (Petrolatum), Paraffinöl, Squalan und Squalen, Polyolefine und hydrogenierte Polyisobutene. Unter den Polyolefinen sind Polydecene die bevorzugten Substanzen.

Besonders vorteilhafte unpolare Lipide im Sinne der vorliegenden Erfindung sind die im folgenden aufgelisteten Substanzen:

| **Hersteller** | **Handelsname** | **INCI-Name** | **Polarit ät [mN/m]** |
|---|---|---|---|
| Total SA | Ecolane 130 | Cycloparaffin | 49,1 |
| Neste PAO N.V. | | Polydecene | 46.7 |
| (Lief. Hansen & Rosenthal) | Nexbase 2006 FG | | |
| Chemische Fabrik Lehrte | Polysynlane | Hydrogenated | 44.7 |
| | | Polyisobutene | |
| Wacker | Wacker Silikonöl AK 50 | Polydimethylsiloxan | 46,5 |
| EC Erdölchemie (Lieferant Bayer AG) | Solvent ICH | Isohexadecane | 43.8 |
| DEA Mineralöl (Lief. Hansen & Rosenthal) | Pionier 2076 | Mineral Oil | 43.7 |
| Tudapetrol | | | |
| DEA Mineralöl (Lief. Hansen & Rosenthal) | Pionier 6301 | Mineral Oil | 43.7 |
| Tudapetrol | | | |
| Wacker | Wacker Silikonöl AK 35 | Polydimethylsiloxan | 42,4 |
| EC Erdölchemie GmbH | Isoeikosan | Isoeikosan | 41.9 |
| Wacker | Wacker Silikonöl AK 20 | Polydimethylsiloxan | 40,9 |
| Condea Chemie | Isofol 1212 Carbonat | | 40,3 |
| Gattefossé | Softcutol O | Ethoxydiglycol Oleate | 40,5 |
| Creaderm | Lipodermanol OL | Decyl Olivate | 40,3 |
| *Henkel* | *Cetiol S* | Dioctylcyclohexane | 39,0 |
| DEA Mineralöl (Lief. Hansen & Rosenthal) | Pionier 2071 | Mineral Oil | 38.3 |
| Tudapetrol | | | |
| WITCO BV | Hydrobrite 1000 PO | Paraffinum Liquidum | 37,6 |
| Goldschmidt | Tegosoft HP | Isocetyl Palmitate | 36,2 |
| Condea Chemie | Isofol Ester 1693 | | 33,5 |
| Condea Chemie | Isofol Ester 1260 | | 33,0 |
| Dow Coming | Dow Coming Fluid 245 | Cyclopentasiloxan | 32,3 |
| Unichema | Prisorine 2036 | Octyl Isostearate | 31.6 |
| Henkel Cognis | Cetiol CC | Dicaprylyl Carbonate | 31,7 |
| ALZO (ROVI) | Dermol 99 | Trimethylhexyl | 31,1 |
| | | Isononanoate | |
| ALZO (ROVI) | Dermol 89 | 2- Ethylhexyl | 31,0 |
| | | Isononanoate | |
| Henkel Cognis | Cetiol OE | Dicaprylyl Ether | 30,9 |
| | *Dihexylcarbonat* | Dihexyl Carbonate | 30,9 |
| Albemarle S.A. | Silkflo 366 NF | Polydecene | 30,1 |
| Unichema | Estol 1540 EHC | Octyl Cocoate | 30.0 |

Es ist jedoch auch vorteilhaft, Gemische aus höher- und niederpolaren Lipiden und dergleichen zu verwenden. So kann die Ölphase vorteilhaft gewählt werden aus der Gruppe der verzweigten und unverzweigten Kohlenwasserstoffe und -wachse, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr, sofern die im Hauptanspruch geforderten Bedingungen eingehalten werden.

Erfindungsgemäß vorteilhaft zu verwendende Fett- und/oder Wachskomponenten können aus der Gruppe der pflanzlichen Wachse, tierischen Wachse, Mineralwachse und petrochemischen Wachse gewählt werden. Erfindungsgemäß günstig sind beispielsweise Candelillawachs, Carnaubawachs, Japanwachs, Espartograswachs, Korkwachs, Guarumawachs, Reiskeimölwachs, Zuckerrohrwachs, Beerenwachs, Ouricurywachs, Montan-wachs, Jojobawachs, Shea Butter, Bienenwachs, Schellackwachs, Walrat, Lanolin (Wollwachs), Bürzelfett, Ceresin, Ozokerit (Erdwachs), Paraffinwachse und Mikrowachse, sofern die im Hauptanspruch geforderten Bedingungen eingehalten werden.

Weitere vorteilhafte Fett- und/oder Wachskomponenten sind chemisch modifzierte Wachse und synthetische Wachse, wie beispielsweise die unter den Handelsbezeichnungen Syncrowax HRC (Glyceryltribehenat), und Syncrowax AW 1C (C₁₈₋₃₆ -Fettsäure) bei der CRODA GmbH erhältlichen sowie Montanesterwachse, Sasolwachse, hydrierte Jojobawachse, synthetische oder modifizierte Bienenwachse (z. B. Dimethicon Copolyol Bienenwachs und/oder C₃₀₋₅₀ -Alkyl Bienenwachs), Polyalkylenwachse, Polyethylenglykolwachse, aber auch chemisch modifzierte Fette, wie z. B. hydrierte Pflanzenöle (beispielsweise hydriertes Ricinusöl und/oder hydrierte Cocosfettglyceride), Triglyceride, wie beispielsweise Trihydroxystearin, Fettsäuren, Fettsäureester und Glykolester, wie beispielsweise C₂₀₋₄₀-Alkylstearat, C₂₀₋₄₀-Alkylhydroxystearoylstearat und/oder Glykolmontanat. Weiter vorteilhaft sind auch bestimmte Organosiliciumverbindungen, die ähnliche physikalische Eigenschaften aufweisen wie die genannten Fett- und/oder Wachskomponenten, wie beispielsweise Stearoxytrimethylsilan sofern die im Hauptanspruch geforderten Bedingungen eingehalten werden.

Erfindungsgemäß können die Fett- und/oder Wachskomponenten sowohl einzeln als auch im Gemisch vorliegen. Auch beliebige Abmischungen solcher Öl- und Wachskomponenten sind vorteilhaft im Sinne der vorliegenden Erfindung einzusetzen.

Vorteilhaft wird die Ölphase gewählt aus der Gruppe 2-Ethylhexylisostearat, Isotridecylisononanoat, Butylen Glycol Dicaprylat/Dicaprat, 2-Ethylhexylcocoat, C₁₂₋₁₅-Alkylbenzoat, Capryl-Caprinsäure-triglycerid, Dicaprylylether sofern die im Hauptanspruch geforderten Bedingungen eingehalten werden.

Besonders vorteilhaft sind Mischungen aus, Capryl-Caprinsäure-triglycerid, Dicaprylylether, Dicaprylyl Carbonat, Cocoglyceriden, oder Mischungen aus C₁₂₋₁₅-Alkybenzoat und 2-Ethylhexylisostearat, Mischungen aus C₁₂₋₁₅-Alkybenzoat und Butylen Glycol Dicaprylat/Dicaprat sowie Mischungen aus C₁₂₋₁₅-Alkybenzoat, 2-Ethylhexylisostearat und Isotridecylisononanoat sofern die im Hauptanspruch geforderten Bedingungen eingehalten werden.

Von den Kohlenwasserstoffen sind Paraffinöl, Cycloparaffin, Squalan, Squalen, hydriertes Polyisobuten bzw. Polydecen vorteilhaft im Sinne der vorliegenden Erfindung zu verwenden. sofern die im Hauptanspruch geforderten Bedingungen eingehalten werden.

### Silikone

Es kann ebenfalls vorteilhaft sein, die Ölphase der erfindungsgemäßen Zubereitungen teilweise oder vollständig aus der Gruppe der cyclischen und/oder linearen Silicone zu wählen, welche im Rahmen der vorliegenden Offenbarung auch als "Siliconöle" bezeichnet werden. Solche Silicone oder Siliconöle können als Monomere vorliegen, welche in der Regel durch Strukturelemente charakterisiert sind, wie folgt:

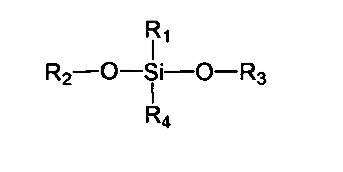

Silikonöle sind hochmolekulare synthetische polymere Verbindungen, in denen Silicium-Atome über Sauerstoff-Atome ketten- und/oder netzartig verknüpft und die restlichen Valenzen des Siliciums durch Kohlenwasserstoff-Reste (meist Methyl-, seltener Ethyl-, Propyl-, Phenyl-Gruppen u. a.) abgesättigt sind.

Als erfindungsgemäß vorteilhaft einzusetzenden linearen Silicone mit mehreren Siloxyleinheiten werden im allgemeinen durch Strukturelemente charakterisiert wie folgt: 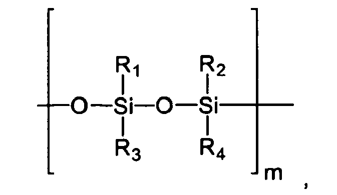 wobei die Siliciumatome mit gleichen oder unterschiedlichen Alkylresten und/oder Arylresten substituiert werden können, welche hier verallgemeinernd durch die Reste R₁ - R₄ dargestellt sind (will sagen, daß die Anzahl der unterschiedlichen Reste nicht notwendig auf bis zu 4 beschränkt ist). m kann dabei Werte von 2 - 200.000 annehmen.

Systematisch werden die linearen Silikonöle als Polyorganosiloxane bezeichnet; die methylsubstituierten Polyorganosiloxane, welche die mengenmäßig bedeutendsten

Verbindungen dieser Gruppe darstellen und sich durch die folgende Strukturformel auszeichnen 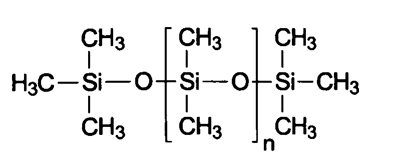 werden auch als Polydimethylsiloxan bzw. Dimethicon (INCI) bezeichnet. Dimethicone gibt es in verschiedenen Kettenlängen bzw. mit verschiedenen Molekulargewichten. Dimethicone unterschiedlicher Kettenlänge und Phenyltrimethicone sind besonders vorteilhafte lineare Silikonöle im Sinne der vorliegenden Erfindung.

Besonders vorteilhafte Polyorganosiloxane im Sinne der vorliegenden Erfindung sind ferner beispielsweise Dimethylpolysiloxane [Poly(dimethylsiloxan)], welche z. B. unter den Handelsbezeichnungen ABIL 10 bis 10 000 bei Th. Goldschmidt erhältlich sind. Ferner vorteilhaft sind Phenylmethylpolysiloxane (INCI: Phenyl Dimethicone, Phenyl Trimethicone), cyclische Silicone (Octamethylcyclotetrasiloxan bzw. Decamethylcydopentasiloxan), welche nach INCI auch als Cyclomethicone bezeichnet werden, aminomodifizierte Silicone (INCI: Amodimethicone) und Siliconwachse, z. B. Polysiloxan-Polyalkylen-Copolymere (INCI: Stearyl Dimethicone und Cetyl Dimethicone) und Dialkoxydimethylpolysiloxane (Stearoxy Dimethicone und Behenoxy Stearyl Dimethicone), welche als verschiedene Abil-Wax-Typen bei Th. Goldschmidt erhältlich sind.

Besonders vorteilhaft im Sinne der vorliegenden Erfindung sind ferner die im folgenden aufgelisteten Silikonöle:

| **Hersteller** | **Handelsname** | **INCI-Name** | **Polarität [mN/m]** |
|---|---|---|---|
| Wacker | Wacker Silikonöl AK 100 | Polydimethylsiloxan | 26,9 |
| Wacker | Wacker Silikonöl AK 50 | Polydimethylsiloxan | 46,5 |

| **Hersteller** | **Handelsname** | **INCI-Name** | **Polarität** |
|---|---|---|---|
| Wacker | Wacker Silikonöl AK 35 | Polydimethylsiloxan | 42,4 |
| Wacker | Wacker Silikonöl AK 20 | Polydimethylsiloxan | 40,9 |
| Dow Coming | Dow Coming Fluid 245 | Cyclopentasiloxan | 32,3 |
| Dow Coming | Dow Coming Fluid 345 | Cyclomethicone | 28,5 |

Erfindungsgemäß vorteilhaft einzusetzende cyclische Silicone werden im allgemeinen durch Strukturelemente charakterisiert, wie folgt 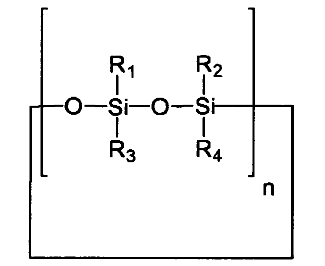 wobei die Siliciumatome mit gleichen oder unterschiedlichen Alkylresten und/oder Arylresten substituiert werden können, welche hier verallgemeinernd durch die Reste R₁ - R₄ dargestellt sind (will sagen, daß die Anzahl der unterschiedlichen Reste nicht notwendig auf bis zu 4 beschränkt ist). n kann dabei Werte von 3/2 bis 20 annehmen. Gebrochene Werte für n berücksichtigen, daß ungeradzahlige Anzahlen von Siloxylgruppen im Cyclus vorhanden sein können.

Besonders vorteilhafte cyclische Silikonöle im Sinne der vorliegenden Erfindung sind Cyclomethicone, insbesondere Cyclomethicone D5 und/oder Cyclomethicone D6.

Vorteilhafte Silkonöle bzw. Silikonwachse im Sinne der vorliegenden Erfindung sind cyclische und/oder lineare Silikonöle und Silikonwachse.

Es ist besonders vorteilhaft im Sinne der vorliegenden Erfindung das Verhältnis von Lipiden zu Silikonölen in etwa wie 1 : 1 (allgemein x : y) zu wählen.

Vorteilhaft wird Phenyltrimethicon als Siliconöl gewählt. Auch andere Silikonöle, beispielsweise Dimethicon, Phenyldimethicon, Cyclomethicon (Octamethylcydotetrasiloxan) beispielsweise Hexamethylcyclotrisiloxan, Polydimethylsiloxan, Poly(methyphenylsiloxan), Cetyldimethicon, Behenoxydimethicon sind vorteilhaft im Sinne der vorliegenden Erfindung zu verwenden.

Vorteilhaft sind ferner Mischungen aus Cyclomethicon und Isotridecylisononanoat, sowie solche aus Cyclomethicon und 2-Ethylhexylisostearat.

Es ist aber auch vorteilhaft, Silikonöle ähnlicher Konstitution wie der vorstehend bezeichneten Verbindungen zu wählen, deren organische Seitenketten derivatisiert, beispielsweise polyethoxyliert und/oder polypropoxyliert sind. Dazu zählen beispielsweise Poly-siloxan-polyalkyl-polyether-copolymere wie das Cetyl-Dimethicon-Copolyol sowie das Cetyl-Dimethicon-Copolyol (und) Polyglyceryl-4-Isostearat (und) Hexyllaurat.

Die erfindungsgemäßen Emulsionen können vorteilhaft anfeuchtende bzw. feuchthaltende Mittel (sogenannte Moisturizer) enthalten. Vorteilhafte feuchthaltende Mittel im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist.

Vorteilhaft werden die Antioxidantien gewählt aus der Gruppe bestehend aus Aminosäuren (z.B. Glycin, Lysin, Arginin, Cystein, Histidin, Tyrosin, Tryptophan) und deren Derivate (als Salz-, Ester-, Ether-, Zucker-, Nukleotid-, Nukleosid-, Peptid- und Lipid-Verbindung), Imidazole (z.B. Urocaninsäure) und deren Derivate (als Salz-, Ester-, Ether-, Zucker-, Nukleotid-, Nukleosid-, Peptid- und/oder Lipid-Verbidung), Peptide wie D,L-Carnosin, D-Carnosin, L-Carnosin, Anserin und deren Derivate (z.B. als Salz-, Ester-, Ether-, Zucker-, Thiol-, Nukleotid-, Nukleosid-, Peptid- und Lipid-Verbidung), Carotinoide, Carotine (z.B. α-Carotin, β-Carotin, ψ-Lycopin, Phytoen, ) und deren Derivate (z. B. als Salz-, Ester-, Ether-, Zucker-, Nukleotid-, Nukleosid-, Peptid- und/oder Lipid-Verbidung), Chlorogensäure und deren Derivate (als Salz-, Ester-, Ether-, Zucker-, Thiol-, Nukleotid-, Nukleosid-, Peptid- und/oder Lipid-Verbidung), Aurothioglucose, Propylthiouracil und andere Thiole (z.B. Thioredoxin, Liponsäure, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (als Salz-, Ester-, Ether-, Zucker-, Thiol-, Nukleotid-, Nukleosid-, Peptid- und/oder Lipid-Verbidung) sowie Sulfoximinverbindungen (z.B. Homocysteinsulfoximin, Buthioninsulfone, Penta-, Hexa-, Heptathioninsulfoximin) in sehr geringen verträglichen Dosierungen (z.B. pmol bis µmol/kg). Ferner (Metall)-Chelatoren (z.B. Apoferritin, Desferral, Lactoferrin, α-Hydroxyfettsäuren, Palmitinsäure, Phytinsäure) und deren Derivate (als Salz-, Ester-, Ether-, Zucker-, Thiol-, Nukleotid-, Nukleosid-, Peptid- und/oder Lipid-Verbidung), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Furfurylidensorbitol und dessen Derivate, Ubichinon, Ubichinol, Plastochinon und deren Derivate (als Salz-, Ester-, Ether-, Zucker-, Thiol-, Nukleotid-, Nukleosid-, Peptid- und Lipid-Verbidung), Vitamin C und Derivate (z.B. Ascorbylpalmitat, Mg-Ascorbylphosphat, Ascorbylacetat), Tocopherole und Derivate (z.B. Vitamin-E-acetat), sowie Phenolische Verbindungen und Pflanzenextrakte, diese enthaltend, wie z. B. Flavonoide (z. B. Glycosylrutin, Ferulasäure, Kaffeesäure), Furfurylidenglucitol, Butylhydroxytoluol, Butylhydroxyanisol, Nordihydroguajakharzsäure, Nordihydroguajaretsäure, Trihydroxybutyrophenon und deren Derivate (als Salz-, Ester-, Ether-, Zucker-, Nukleotid-, Nukleosid-, Peptid- und Lipid-Verbidung). Harnsäure und deren Derivate, Mannose und deren Derivate (als Salz-, Ester-, Ether-, Zucker-, Thiol-, Nukleotid-, Nukleosid-, Peptid- und Lipid-Verbidung). Zink und dessen Derivate (z.B. ZnO, ZnSO₄), Selen und dessen Derivate (z.B. Selenmethionin, Ebselen), Stilbene und deren Derivate (z.B. Stilbenoxid, Trans-Stilbenoxid) und die erfindungsgemäß geeigneten Derivate (als Salz-, Ester-, Ether-, Zucker-, Thiol-, Nukleotid-, Nukleosid-, Peptid- und/oder Lipid-Verbidung) dieser genannten Wirkstoffe.

Weitere vorteilhafte Wirkstoffe im Sinne der vorliegenden Erfindung sind natürliche Wirkstoffe und/oder deren Derivate, wie z. B. alpha-Liponsäure, Phytoen, D-Biotin, Coenzym Q10, alpha-Glucosylrutin, Carnitin, Carnosin, Vitamin A und seine Derivate, natürliche und/oder synthetische Isoflavonoide, Kreatin, Kreatinin, Taurin und/oder β-Alanin.

Erfindungsgemäße Rezepturen, welche z. B. bekannte Antifaltenwirkstoffe wie Flavonglycoside (insbesondere α-Glycosylrutin), Coenzym Q10, Vitamin E und/oder Derivate und dergleichen enthalten, eignen sich insbesondere vorteilhaft zur Prophylaxe und Behandlung kosmetischer oder dermatologischer Hautveränderungen, wie sie z. B. bei der Hautalterung auftreten (wie beispielsweise Trockenheit, Rauhigkeit und Ausbildung von Trockenheitsfältchen, Juckreiz, verminderte Rückfettung (z. B. nach dem Waschen), sichtbare Gefäßerweiterungen (Teleangiektasien, Cuperosis), Schlaffheit und Ausbildung von Falten und Fältchen, lokale Hyper-, Hypo- und Fehlpigmentierungen (z. B. Altersflecken), vergrößerte Anfälligkeit gegenüber mechanischem Stress (z. B. Rissigkeit) und dergleichen). Weiterhin vorteilhaft eignen sie sich gegen das Erscheinungsbild der trockenen bzw. rauhen Haut.

In die erfindungsgemäßen Zubereitungen können aber auch andere pharmazeutisch oder dermatologisch wirkende Substanzen wie beispielsweise die Haut beruhigende und pflegende Substanzen eingearbeitet sein. Hierzu zählen beispielsweise Panthenol, Niacinamid, Allantoin, Tannin, Antihistaminika, Antiphlogistika, Glucocorticoide (z.B. Hydrocortison) sowie Pflanzenwirkstoffe wie Azulen und Bisabolol, Glycyrrhizin, Hamamelin und Pflanzenextrakte wie Kamille, aloe vera, Hamazelis, Süßholzwurzel. Auch die Vitamin D₃-analoga Tacalcitol, Calcipotriol, Tacalcitol, Colecalciferol sowie Calcitrol (Vitamin D₃) und/oder Fumarsäureester können erfolgreich in die Zubereitungen eingearbeitet werden.

Die erfindungsgemäßen Zubereitungen können einen oder mehrere dieser Wirkstoffe (Moisturizer, Antioxidantien, sonstige Wirkstoffe) jeweils in einer Konzentration von 0,001 bis 30 Gewichts-%, bezogen auf das Gesamtgewicht der Zubereitung enthalten.

Es ist erfindungsgemäß vorteilhaft, wenn die erfindungsgemäßen Emulsionen einen Gehalt an UV-Schutzsubstanzen enthalten. So werden z. B. in Tagescrèmes oder Makeup-Produkten gewöhnlich UV-A- bzw. UV-B-Filtersubstanzen eingearbeitet. Auch stellen UV-Schutzsubstanzen, ebenso wie Antioxidantien und, gewünschtenfalls, Konservierungsstoffe, einen wirksamen Schutz der Zubereitungen selbst gegen Verderb dar. Günstig sind ferner kosmetische und dermatologische Zubereitungen, die in der Form eines Sonnenschutzmittels vorliegen.

Dementsprechend enthalten die Emulsionen im Sinne der vorliegenden Erfindung vorzugsweise mindestens eine UV-A- und/oder UV-B-Filtersubstanz. Die Formulierungen können, obgleich nicht notwendig, gegebenenfalls auch ein oder mehrere organische und/oder anorganische Pigmente als UV-Filtersubstanzen enthalten, welche in der Wasser- und/oder der Ölphase vorliegen können.

Bevorzugte anorganische Pigmente sind Metalloxide und/oder andere in Wasser schwerlösliche oder unlösliche Metallverbindungen, insbesondere Oxide des Titans (TiO₂), Zinks (ZnO), Eisens (z. B. Fe₂O₃), Zirkoniums (ZrO₂), Siliciums (SiO₂), Mangans (z. B. MnO), Aluminiums (Al₂O₃), Cers (z. B. Ce₂O₃), Mischoxide der entsprechenden Metalle sowie Abmischungen aus solchen Oxiden, sowie das Sulfat des Bariums (BaSO₄).

Die Titandioxid- Pigmente können sowohl in der Kristallmodifikation Rutil als auch Anatas vorliegen und können im Sinne der vorliegenden Erfindung vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, daß die Pigmente nach an sich bekannten Verfahren mit einer dünnen hydrophilen und/oder hydrophoben anorganischen und/oder organischen Schicht versehen werden. Die verschiedenen Oberflächenbeschichtung können im Sinne der vorliegenden Erfindung auch Wasser enthalten.

Beschriebene beschichtete und unbeschichtete Titandioxide können im Sinne vorliegender Erfindung auch in Form kommerziell erhältlicher öliger oder wäßriger Vordispersionen zur Anwendung kommen. Diesen Vordispersionen können vorteilhaft Dispergierhilfmittel und/oder Solubilisationsvermittler zugesetzt sein.

Die erfindungsgemäßen Titandioxide zeichnen sich durch eine Primärpartikelgröße zwischen 10 nm bis 150 nm aus.

| **Handelsname** | **Coating** | **zusätzliche Bestandteile der Vordispersion** | **Hersteller** |
|---|---|---|---|
| MT-100TV | Aluminiumhydroxid | - | Tayca Corporation |
| | Stearinsäure | | |
| MT-100Z | Aluminiumhydroxid | - | Tayca Corporation |
| | Stearinsäure | | |
| MT-100F | Stearinsäure | - | Tayca Corporation |
| | Eisenoxid | | |
| MT-500SAS | Alumina, Silica | - | Tayca Corporation |
| | Silikon | | |
| MT-100AQ | Silica | - | Tayca Corporation |
| | Aluminiumhydroxid | | |
| | Alginsäure | | |
| Eusolex T-2000 | Alumina | - | Merck KgaA |
| | Simethicone | | |
| Eosolex TS | Alumina, Stearinsäure | - | Merck KgaA |
| Titandioxid P25 | None | - | Degussa |
| Titandioxid T805 | Octyltrimethylsilan | - | Degussa |
| (Uvinul TiO₂) | | | |
| UV-Titan X170 | Alumina | - | Kemira |
| | Dimethicone | | |
| UV-Titan X161 | Alumina, Silica | - | Kemira |
| | Stearinsäure | | |
| Tioveil AQ 10PG | Alumina | Wasser | Solaveil |
| | Silica | Propylenglycol | Uniquema |
| Mirasun TiW 60 | Alumina | Wasser | Rhone-Poulenc |
| | Silica | | |

Im Sinne der vorliegenden Erfindung sind besonders bevorzugte Titandioxide das MT-100 Z und MT-100 TV von Tayca Corporation, Eusolex T-2000 und Eusolex TS von Merck und das Titandioxid T 805 von Degussa.

Zinkoxide können im Sinne der vorliegenden Erfindung auch in Form kommerziell erhältlicher öliger oder wäßriger Vordispersionen zur Anwendung kommen. Erfindungsgemäß geeignete Zinkoxidpartikel und Vordispersionen von Zinkoxidpartikeln zeichnen sich durch eine Primärpartikelgröße von < 300 nm aus und sind unter folgenden Handelsbezeichnungen bei den aufgeführten Firmen erhältlich:

| **Handelsname** | **Coating** | **Hersteller** |
|---|---|---|
| Z- Cote HP1 | 2% Dimethicone | BASF |
| Z- Cote | / | BASF |
| ZnO NDM | 5% Dimethicone | H&R |
| MZ 707M | 7% Dimethicone | M. Tayca Corp. |
| Nanox 500 | / | Elementis |
| ZnO Neutral | / | H&R |

Besonderes bevorzugte Zinkoxide im Sinne der Erfindung sind das Z-Cote HP1 von der Firma BASF und das Zinkoxid NDM von der Firma Haarmann & Reimer.

Die Gesamtmenge an einem oder mehreren anorganischen Pigmenten in der fertigen kosmetischen Zubereitung wird vorteilhaft aus dem Bereich 0,1 Gew.-% bis 25 Gew.-% gewählt, vorzugsweise 0,5 Gew.-% bis 18 Gew.-%.

Vorteilhaftes organisches Pigment im Sinne der vorliegenden Erfindung ist das 2,2'-Methylen-bis-(6-(2H-benzotriazol-2-yl)-4-(1,1,3,3-tetramethylbutyl)-phenol) [INCI: Bisoctyltriazol], welches unter der Handelsbezeichnung Tinosorb® M bei der CIBA-Chemikalien GmbH erhältlich ist.

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol® 1789 und von Merck unter der Handelsbezeichnung Eusolex® 9020 verkauft wird.

Weitere vorteilhafte UV-A-Filtersubstanzen sind die Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz mit der INCI-Bezeichnung Bisimidazylate, welches beispielsweise unter der Handelsbezeichnung Neo Heliopan AP bei Haarmann & Reimer erhältlich ist.

Ferner vorteilhaft sind das 1,4-di(2-oxo-10-Sulfo-3-bornylidenmethyl)-Benzol und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird.

Weitere vorteilhafte UV-A-Filtersubstanzen sind Hydroxybenzophenone, die sich durch die folgende Strukturformel auszeichnen: 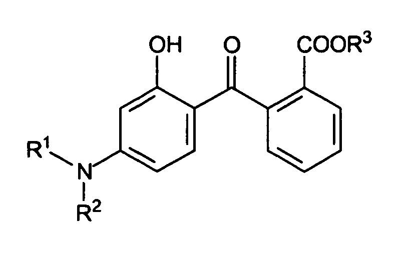 worin
- R¹ und R² unabhängig voneinander Wasserstoff, C₁-C₂₀-Alkyl, C₃-C₁₀-Cycloalkyl oder C₃-C₁₀-Cycloalkenyl bedeuten, wobei die Substituenten R¹ und R² gemeinsam mit dem Stickstoffatom, an das sie gebunden sind, einen 5- oder 6-Ring bilden können und
- R³ einen C₁-C₂₀-Alkyl Rest bedeutet.

Ein besonders vorteilhaftes Hydroxybenzophenon im Sinne der vorliegenden Erfindung ist der 2-(4'-Diethylamino-2'-hydoxybenzoyl)-benzoesäurehexylester (auch: Aminobenzophenon), welcher unter dem Handelsnamen Uvinul A Plus bei der Fa. BASF erhältlich ist.

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Bis-Resorcinyltriazinderivate mit der folgenden Struktur: 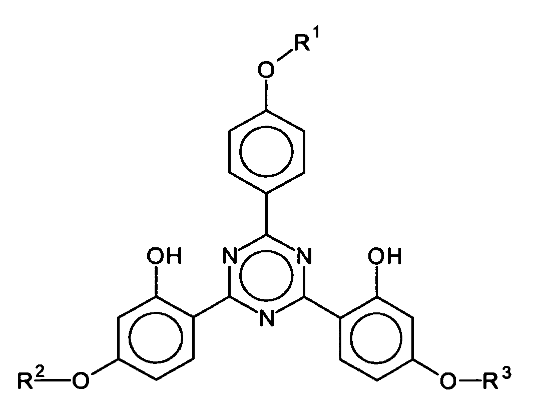 wobei R¹, R² und R³ unabhängig voneinander gewählt werden aus der Gruppe der verzweigten und unverzweigten Alkylgruppen mit 1 bis 10 Kohlenstoffatomen bzw. ein einzelnes Wasserstoffatom darstellen. Insbesondere bevorzugt sind das 2,4-Bis-{[4-(2-Ethyl-hexyloxy)-2-hydroxy]-phenyl}-6-(4-methoxyphenyl)-1,3,5-triazin (INCI: Aniso Triazin), welches unter der Handelsbezeichnung Tinosorb® S bei der CIBA-Chemikalien GmbH erhältlich ist.

Besonders vorteilhafte Zubereitungen im Sinne der vorliegenden Erfindung, die sich durch einen hohen bzw. sehr hohen UV-A-Schutz auszeichnen, enthalten bevorzugt mehrere UV-A- und/oder Breitbandfilter, insbesondere Dibenzoylmethanderivate [beispielsweise das 4-(tert.-Butyl)-4'-methoxydibenzoylmethan], Benzotriazolderivate [beispielsweise das 2,2'-Methylen-bis-(6-(2H-benzotriazol-2-yl)-4-(1,1,3,3-tetramethylbutyl)-phenol)], Phenylen-1,4-bis-(2-benzimidazyl)-3,3'-5,5'-tetrasulfonsäure und/oder ihre Salze, das 1,4-di(2-oxo-10-Sulfo-3-bornylidenmethyl)-Benzol und/oder dessen Salze und/oder das 2,4-Bis-{[4-(2-Ethyl-hexyloxy)-2-hydroxy]-phenyl}-6-(4-methoxyphenyl)-1,3,5-triazin, jeweils einzeln oder in beliebigen Kombinationen miteinander.

Auch andere UV-Filtersubstanzen, welche das Strukturmotiv 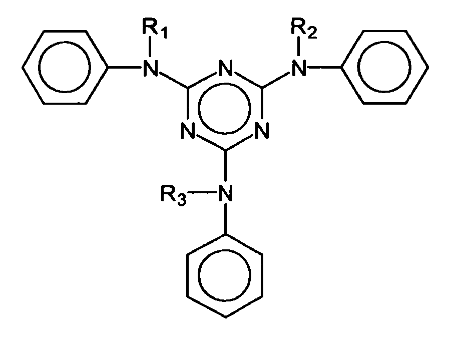 aufweisen, sind vorteilhafte UV-Filtersubstanzen im Sinne der vorliegenden Erfindung, beispielsweise die in der Europäischen Offenlegungsschrift EP 570 838 A1 beschriebenen s-Triazinderivate, deren chemische Struktur durch die generische Formel 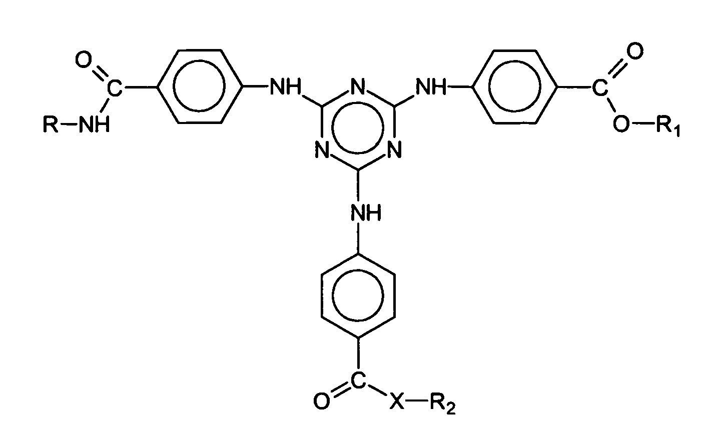 wiedergegeben wird, wobei
- R: einen verzweigten oder unverzweigten C₁-C₁₈-Alkylrest, einen C₅-C₁₂-Cycloalkyl- rest, gegebenenfalls substituiert mit einer oder mehreren C₁-C₄- Alkylgruppen, darstellt,
- X: ein Sauerstoffatom oder eine NH-Gruppe darstellt,
- R₁: einen verzweigten oder unverzweigten C₁-C₁₈-Alkylrest, einen C₅-C₁₂-Cycloalkyl- rest, gegebenenfalls substituiert mit einer oder mehreren C₁-C₄- Alkylgruppen, oder ein Wasserstoffatom, ein Alkalimetallatom, eine Ammoniumgruppe oder eine Gruppe der Formel 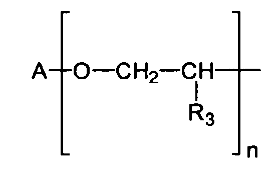
bedeutet, in welcher
- A: einen verzweigten oder unverzweigten C₁-C₁₈-Alkylrest, einen C₅-C₁₂-Cy- cloalkyl- oder Arylrest darstellt, gegebenenfalls substituiert mit einer oder mehreren C₁-C₄- Alkylgruppen,
- R₃: ein Wasserstoffatom oder eine Methylgruppe darstellt,
- n: eine Zahl von 1 bis 10 darstellt,

- R₂: einen verzweigten oder unverzweigten C₁-C₁₈-Alkylrest, einen C₅-C₁₂-Cycloalkyl- rest, gegebenenfalls substituiert mit einer oder mehreren C₁-C₄- Alkylgruppen, darstellt, wenn X die NH-Gruppe darstellt, und einen verzweigten oder unverzweigten C₁-C₁₈-Alkylrest, einen C₅-C₁₂-Cycloalkyl- rest, gegebenenfalls substituiert mit einer oder mehreren C₁-C₄- Alkylgruppen, oder ein Wasserstoffatom, ein Alkalimetallatom, eine Ammoniumgruppe oder eine Gruppe der Formel 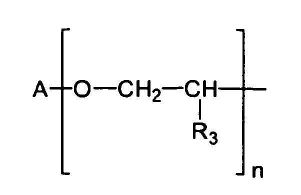
bedeutet, in welcher
- A: einen verzweigten oder unverzweigten C₁-C₁₈-Alkylrest, einen C₅-C₁₂- Cycloalkyl- oder Arylrest darstellt, gegebenenfalls substituiert mit einer oder mehreren C₁-C₄- Alkylgruppen,
- R₃: ein Wasserstoffatom oder eine Methylgruppe darstellt,
- n: eine Zahl von 1 bis 10 darstellt,
wenn X ein Sauerstoffatom darstellt.

Besonders bevorzugte UV-Filtersubstanz im Sinne der vorliegenden Erfindung ist ferner ein unsymmetrisch substituiertes s-Triazin, welches im Folgenden auch als Dioctylbutylamidotriazon (INCI: Dioctylbutamidotriazone) bezeichnet wird und unter der Handelsbezeichnung UVASORB HEB bei Sigma 3V erhältlich ist.

Vorteilhaft im Sinne der vorliegenden Erfindung ist auch ein symmetrisch substituiertes s-Triazin, das 4,4',4"-(1,3,5-Triazin-2,4,6-triyltriimino)-tris-benzoesäure-tris(2-ethylhexylester), synonym: 2,4,6-Tris-[anilino-(p-carbo-2'-ethyl-1'-hexyloxy)]-1,3,5-triazin (INCI: Octyl Triazone), welches von der BASF Aktiengesellschaft unter der Warenbezeichnung UVINUL® T 150 vertrieben wird.

Auch in der Europäischen Offenlegungsschrift 775 698 werden bevorzugt einzusetzende Bis-Resorcinyltriazinderivate beschrieben, deren chemische Struktur durch die generische Formel 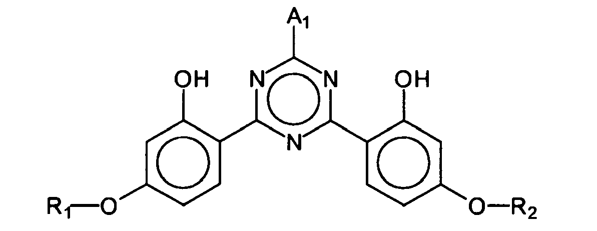 wiedergegeben wird, wobei R₁ , R₂ und A₁ verschiedenste organische Reste repräsentieren.

Vorteilhaft im Sinne der vorliegenden Erfindung sind ferner das 2,4-Bis-{[4-(3-sulfonato)-2-hydroxy-propyloxy)-2-hydroxy]-phenyl}-6-(4-methoxyphenyl)-1,3,5-triazin Natriumsalz, das 2,4-Bis-{[4-(3-(2-Propyloxy)-2-hydroxy-propyloxy)-2-hydroxy]-phenyl}-6-(4-methoxyphenyl)-1,3,5-triazin, das 2,4-Bis-{[4-(2-ethyl-hexyloxy)-2-hydroxy]-phenyl}-6-[4-(2-methoxyethyl-carboxyl)-phenylamino]-1,3,5-triazin, das 2,4-Bis-{[4-(3-(2-propyloxy)-2-hydroxy-propyloxy)-2-hydroxy]-phenyl}-6-[4-(2-ethyl-carboxyl)-phenylamino]-1,3,5-triazin, das 2,4-Bis-{[4-(2-ethyl-hexyloxy)-2-hydroxy]-phenyl}-6-(1-methyl-pyrrol-2-yl)-1,3,5-triazin, das 2,4-Bis-{[4-tris(trimethylsiloxy-silylpropyloxy)-2-hydroxy]-phenyl}-6-(4-methoxyphenyl)-1,3,5-triazin, das 2,4-Bis-{[4-(2"-methylpropenyloxy)-2-hydroxy]-phenyl}-6-(4-methoxyphenyl)-1,3,5-triazin und das 2,4-Bis-{[4-(1',1',1',3',5',5',5'-Heptamethylsiloxy-2"-methyl-propyloxy)-2-hydroxy]-phenyl}-6-(4-methoxyphenyl)-1,3,5-triazin.

Ein vorteilhafter Breitbandfilter im Sinne der vorliegenden Erfindung ist das 2,2'-Methylen-bis-(6-(2H-benzotriazol-2-yl)-4-(1,1,3,3-tetramethylbutyl)-phenol), welches unter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane.

Die UV-B- und/oder Breitband-Filter können öllöslich oder wasserlöslich sein. Vorteilhafte öllösliche UV-B- und/oder Breitband-Filtersubstanzen sind z. B
■ 3-Benzylidencampher-Derivate, vorzugsweise 3-(4-Methylbenzyliden)campher, 3-Benzylidencampher;
■ 4-Aminobenzoesäure-Derivate, vorzugsweise 4-(Dimethylamino)-benzoesäure(2-ethylhexyl)ester, 4-(Dimethylamino)benzoesäureamylester;
■ 2,4,6-Trianilino-(p-carbo-2'-ethyl-1'-hexyloxy)-1,3,5-triazin;
■ Ester der Benzalmalonsäure, vorzugsweise 4-Methoxybenzalmalonsäuredi(2-ethylhexyl)ester;
■ Ester der Zimtsäure, vorzugsweise 4-Methoxyzimtsäure(2-ethylhexyl)ester, 4-Methoxyzimtsäureisopentylester;
■ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon
■ sowie an Polymere gebundene UV-Filter.

Vorteilhafte wasserlösliche UV-B- und/oder Breitband-Filtersubstanzen sind z. B.:
■ Salze der 2-Phenylbenzimidazol-5-sulfonsäure, wie ihr Natrium-, Kalium- oder ihr Triethanolammonium-Salz, sowie die Sulfonsäure selbst;
■ Sulfonsäure-Derivate des 3-Benzylidencamphers, wie z. B. 4-(2-Oxo-3-bornyliden-methyl)benzolsulfonsäure, 2-Methyl-5-(2-oxo-3-bornylidenmethyl)sulfonsäure und deren Salze.

Besonders vorteilhafte bei Raumtemperatur flüssige UV-Filtersubstanzen im Sinne der vorliegenden Erfindung sind Homomenthylsalicylat (INCI: Homosalate), 2-Ethylhexyl-2-hydroxybenzoat (2-Ethylhexylsalicylat, Octylsalicylat, INCI: Octyl Salicylate), 4-Isopropylbenzylsalicylat und Ester der Zimtsäure, vorzugsweise 4-Methoxyzimtsäure(2-ethylhexyl)ester (2-Ethylhexyl-4-methoxycinnamat, INCI: Octyl Methoxycinnamate) und 4-Methoxyzimtsäureisopentylester (Isopentyl-4-methoxycinnamat, INCI: Isoamyl p-Methoxycinnamate), 3-(4-(2,2-bis Ethoxycarbonylvinyl)-phenoxy)propenyl)-methoxysiloxan/ Dimethylsiloxan - Copolymer (INCI: Dimethicodiethylbenzalmalonat) welches beispielsweise unter der Handelsbezeichnung Parsol® SLX bei Hoffmann La Roche erhältlich ist.

Eine weiterere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul^{®} N 539 erhältlich ist.

Es kann auch von erheblichem Vorteil sein, polymergebundene oder polymere UV-Filtersubstanzen in Zubereitungen gemäß der vorliegenden Erfindung zu verwenden, insbesondere solche, wie sie in der WO-A-92/20690 beschrieben werden.

Die Liste der genannten UV-Filter, die im Sinne der vorliegenden Erfindung eingesetzt werden können, soll selbstverständlich nicht limitierend sein.

Vorteilhaft enthalten die erfindungsgemäßen Zubereitungen die Substanzen, die UV-Strahlung im UV-A- und/oder UV-B-Bereich absorbieren, in einer Gesamtmenge von z. B. 0,1 Gew.-% bis 30 Gew.-%, vorzugsweise 0,5 bis 20 Gew.-%, insbesondere 1,0 bis 15,0 Gew.-%, jeweils bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Erfindungsgemäß vorteilhaft kann die erfindungsgemäße Emulsion Mikropartikel enthalten, deren mittlerer Partikeldurchmesser zwischen 1 nm und 200 nm, besonders vorteilhaft zwischen 5 nm und 100 nm liegt.

Es ist ferner vorteilhaft, die Konzentration aller Partikel größer als 0,1 Gew.-%, besonders vorteilhaft zwischen 0,1 Gew.-% und 30 Gew.-%, bezogen auf das Gesamtgewicht der Zubereitungen, zu wählen.

Im wesentlichen unerheblich für die vorliegende Erfindung ist es, in welcher der gegebenenfalls natürlich vorkommenden Modifikationen die Partikel vorliegen.

Vorzugsweise werden unbehandelte, nahezu reine Pigmentpartikel verwendet, insbesondere solche, welche auch als Farbstoff in der Lebensmittelindustrie und/oder als Absorber von UV-Strahlung in Sonnenschutzmitteln verwendet werden können. Vorteilhaft sind beispielsweise die bei der Firma Merck erhältlichen Zinkoxid-Pigmente sowie solche, die unter den Handelsbezeichnungen Zinkoxid neutral bei Haarmann & Reimer oder NanoX von der Harcros Chemical Group erhältlich sind.

Vorteilhaft sind ferner Pigmente, die oberflächlich wasserabweisend behandelt ("gecoatet") sind. Diese Oberflächenbehandlung kann darin bestehen, daß die Pigmente nach an sich bekannten Verfahren mit einer dünnen hydrophoben Schicht versehen werden.

Besonders vorteilhaft sind TiO₂-Pigmente, beispielsweise die mit Aluminiumstearat beschichteten, unter der Handelsbezeichnung MT 100 T bei der Firma TAYCA erhältlichen.

Eine weitere vorteilhafte Beschichtung der anorganische Pigmente besteht aus Dimethylpolysiloxan (auch: Dimethicon), einem Gemisch vollmethylierter, linearer Siloxanpolymere, die endständig mit Trimethylsiloxy-Einheiten blockiert sind. Besonders vorteilhaft im Sinne der vorliegenden Erfindung sind Zinkoxid-Pigmente, die auf diese Weise beschichtet werden.

Vorteilhaft ist ferner eine Beschichtung der anorganischen Pigmente mit einem Gemisch aus Dimethylpolysiloxan, insbesondere Dimethylpolysiloxan mit einer durchschnittlichen Kettenlänge von 200 bis 350 Dimethylsiloxan-Einheiten, und Silicagel, welches auch als Simethicone bezeichnet wird. Es ist insbesondere von Vorteil, wenn die anorganischen Pigmente zusätzlich mit Aluminiumhydroxid bzw. Aluminiumoxidhydrat (auch: Alumina, CAS-Nr.: 1333-84-2) beschichtet sind. Besonders vorteilhaft sind Titandioxide, die mit Simethicone und Alumina beschichtet sind, wobei die Beschichtung auch Wasser enthalten kann. Ein Beispiel hierfür ist das unter dem Handelsnamen Eusolex T2000 bei der Firma Merck erhältliche Titandioxid.

Vorteilhaft im Sinne der vorliegenden Erfindung ist ferner die Verwendung einer Mischung verschiedener Pigmenttypen sowohl innerhalb eines Kristalls, beispielsweise als Eisenmischoxid, als auch durch Kombination mehrerer Pigmenttypen innerhalb einer Zubereitung.

Vorzugsweise sind ferner Bornitridpartikel, beispielsweise die im folgenden aufgelisteten Bornitride:
- Handelsname: erhältlich bei
- Boron Nitride Powder: Advanced Ceramics
- Boron Nitride Powder: Sintec Keramik
- Ceram Blanche: Kawasaki
- HCST Boron Nitride: Stark
- Très BN^{®}: Carborundum
- Wacker-Bornitrid BNP: Wacker-Chemie

Vorteilhaft ist es, den mittleren Partikeldurchmesser der verwendeten Bornitridpartikel kleiner als 20 µm, besonders vorteilhaft kleiner als 15 µm zu wählen.

Vorteilhaft sind ferner beispielsweise die bei der Firma Carborundum unter der Handelsbezeichnung Très BN^{®} UHP 1106 erhältlichen, mit Dimethicon behandelten Bornitridpartikel.

Vorteilhaft ist ferner eine Beschichtung der Bornitridpartikel mit Polymethylhydrogensiloxan, einem linearen Polysiloxan, welches auch als Methicone bezeichnet wird. Vorteilhafte, mit Methicone behandelte Bornitridpartikel sind beispielsweise die bei der Firma Carborundum unter der Handelsbezeichnung Très BN^{®} UHP 1107 erhältlichen.

Weitere vorteilhafte Pigmente sind mikrofeine Polymerpartikel.

Vorteilhafte mikrofeine Polymerpartikel sind im Sinne der vorliegenden Erfindung beispielsweise Polycarbonate, Polyether, Polyethylen, Polypropylen, Polyvinylchlorid, Polystyrol, Polyamide und dergleichen mehr.

Erfindungsgemäß vorteilhaft sind beispielsweise mikrofeine Polyamid-Partikel, welche unter der Handelsbezeichnung SP-500 bei der Firma TORAY erhältlich sind. Ferner vorteilhaft sind Polyamid 6- (auch: Nylon 6) bzw. Polyamid 12- (auch: Nylon 12) Partikel. Polyamid 6 ist das aus ε-Aminocapronsäure (6-Aminohexansäure) oder ε-Caprolactam aufgebaute Polyamid [Poly(ε-caprolactam)], und Polyamid 12 ist ein Poly(ε-laurinlactam) aus ε-Laurinlactam. Vorteilhaft im Sinne der vorliegenden Erfindung sind beispielsweise Orgasol^{®} 1002 (Polyamid 6) und Orgasol^{®} 2002 (Polyamid 12) von der Firma ELF ATOCHEM.

Es ist insbesondere vorteilhaft, wenngleich nicht zwingend, wenn die verwendeten mikrofeinen Polymerpartikel oberflächlich beschichtet sind. Diese Oberflächenbehandlung kann darin bestehen, daß die Pigmente nach an sich bekannten Verfahren mit einer dünnen hydrophilen Schicht versehen werden. Vorteilhafte Beschichtungen bestehen beispielsweise aus TiO₂, ZrO₂ oder auch weiteren Polymeren.

Besonders vorteilhafte mikrofeine Polymerpartikel im Sinne der vorliegenden Erfindung sind ferner nach dem in der US-Patentschrift 4,898,913 beschriebenen Verfahren zur hydrophilen Beschichtung hydrophober Polymerpartikel erhältlich.

Vorteilhaft in ist es, den mittleren Partikeldurchmesser der verwendeten mikrofeinen Polymerpartikel kleiner als 100 µm, besonders vorteilhaft kleiner als 50 µm zu wählen. Dabei ist es im wesentlichen unerheblich, in welcher Form (Plättchen, Stäbchen, Kügelchen etc.) die verwendeten Polymerpartikel vorliegen.

Desweiteren ist es vorteilhaft, modifizierte Polysaccharide zu verwenden.

Modifizierte Polysaccharide im Sinne der vorliegenden Erfindung sind beispielsweise durch Umsetzung von Stärke mit mono-, bi- oder polyfunktionellen Reagenzien bzw. Oxidations-Mitteln in weitgehend polymeranalog verlaufenden Reaktionen erhältlich.

Solche Reaktionen basieren im wesentlichen auf Umwandlungen der HydroxyGruppen der Polyglucane durch Veretherung, Veresterung oder selektive Oxidation.

Dabei entstehen z. B. sogenannte Stärkeether und Stärkeester der allgemeinen Strukturformel 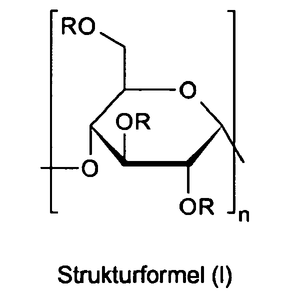 worin R beispielsweise ein Wasserstoff und/oder einen Alkyl- und/oder Aralkylrest (im Fall der Stärkeether) oder ein Wasserstoff und/oder einen organischen und/oder anorganischen Säure-Rest (im Fall der Stärkeester) darstellen kann. Stärkeether und Stärkeester sind vorteilhafte modifizierte Polysaccharide im Sinne der vorliegenden Erfindung.

Besonders vorteilhafte Stärkeether sind z. B. solche, die durch Veretherung von Stärke mit Tetramethylolacetylendiharnstoff erhältlich sind und welche als Amylum non mucilaginosum (nicht quellende Stärke) bezeichnet werden.

Insbesondere vorteilhaft sind ferner Stärkeester und deren Salze, beispielsweise die Natrium- und/oder Aluminiumsalze niedrigsubstituierter Halbester der Stärke, insbesondere Natrium Stärke n-Octenylsuccinat der Strukturformel (I), worin R sich durch die folgende Struktur auszeichnet 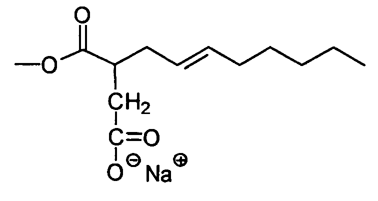 und welches z. B. unter der Handelsbezeichnung Amiogum^{®} 23 bei der Firma CERESTAR erhältlich ist sowie Aluminium Stärke Octenylsuccinate, insbesondere die unter den Handelsbezeichnungen Dry Flo^{®} Elite LL und Dry Flo^{®} PC bei der Firma CERESTAR erhältlichen.

Vorteilhaft ist es, den mittleren Partikeldurchmesser der verwendeten modifizierten Polysaccharide kleiner als 20 µm, besonders vorteilhaft kleiner als 15 µm zu wählen. Die Liste der genannten modifizierten Polysaccharide soll selbstverständlich nicht limitierend sein. Modifizierte Polysaccharide im Sinne der vorliegenden Erfindung sind auf zahlreichen, an sich bekannten Wegen, sowohl chemischer als auch physikalischer Natur erhältlich.

Die Zusammensetzungen enthalten gemäß der Erfindung außer den vorgenannten Substanzen gegebenenfalls die in der Kosmetik üblichen Zusatzstoffe, beispielsweise Parfüm, Farbstoffe, antimikrobielle Stoffe, rückfettende Agentien, Komplexierungs- und Sequestrierungsagentien, Perlglanzagentien, Pflanzenextrakte, Vitamine, Wirkstoffe, Konservierungsmittel, Bakterizide, Tenside, Pigmente, die eine färbende Wirkung haben, Verdickungsmittel, weich machende, anfeuchtende und/oder feucht haltende Substanzen, oder andere übliche Bestandteile einer kosmetischen oder dermatologischen Formulierung wie Alkohole, Polyole, Polymere, Schaumstabilisatoren, Elektrolyte, organische Lösemittel oder Silikonderivate.

Die erfindungsgemäße Zubereitung kann erfindungsgemäß vorteilhaft ein oder mehrere Konservierungsstoffe enthalten. Vorteilhafte Konservierungsstoffe im Sinne der vorliegenden Erfindung sind beispielsweise Formaldehydabspalter (wie z. B. DMDM Hydantoin, welches beispielsweise unter der Handelsbezeichnung Glydant ™ von der Fa. Lonza erhältlich ist), lodopropylbutylcarbamate (z. B. die unter den Handelsbezeichnungen Glycacil-L, Glycacil-S von der Fa. Lonza erhältlichen und/oder Dekaben LMB von Jan Dekker), Parabene (d. h. p-Hydroxybenzoesäurealkylester, wie Methyl-, Ethyl-, Propyl- und/oder Butylparaben), Phenoxyethanol, Ethanol, Benzoesäure und dergleichen mehr. Üblicherweise umfaßt das Konservierungssystem erfindungsgemäß ferner vorteilhaft auch Konservierungshelfer, wie beispielsweise Octoxyglycerin, Glycine Soja etc. Die nachfolgende Tabelle gibt einen Überblick über einige erfindungsgemäß vorteilhafte Konservierungsstoffe:

| | | | |
|---|---|---|---|
| E 200 | Sorbinsäure | E 227 | Calciumhydrogensulfit |
| E 201 | Natriumsorbat | E 228 | Kaliumhydrogensulfit) |
| E 202 | Kaliumsorbat | E 230 | Biphenyl (Diphenyl) |
| E 203 | Calciumsorbat | E 231 | Orthophenylphenol |
| E 210 | Benzoesäure | E 232 | Natriumorthophenylphenolat |
| E 211 | Natriumbenzoat | E 233 | Thiabendazol |
| E 212 | Kaliumbenzoat | E 235 | Natamycin |
| E 213 | Calciumbenzoat | E 236 | Ameisensäure |
| E 214 | p-Hydroxybenzoesäureethylester | E 237 | Natriumformiat |
| E 215 | p-Hydroxybenzoesäureethylester-Na-Salz | E 238 | Calciumformiat |
| E 216 | p-Hydroxybenzoesäure-n-propylester | E 239 | Hexamethylentetramin |
| E 217 | p-Hydroxybenzoesäure-n-propylester-Na-Salz | E 249 | Kaliumnitrit |
| E 218 | p-Hydroxybenzoesäuremethylester | E 250 | Natriumnitrit |
| E 219 | p-Hydroxybenzoesäuremethylester-Na-Salz | E 251 | Natriumnitrat |
| E 220 | Schwefeldioxid | E 252 | Kaliumnitrat |
| E 221 | Natriumsulfit | E 280 | Propionsäure |
| E 222 | Natriumyhdrogensulfit | E 281 | Natriumpropionat |
| E 223 | Natriumdisulfit | E 282 | Calciumpropionat |
| E 224 | Kaliumdisulfit | E 283 | Kaliumpropionat |
| E 226 | Calciumsulfit | E 290 | Kohlendioxid |

Ferner vorteilhaft sind in der Kosmetik gebräuchliche Konservierungsmittel oder Konservierungshilfsstoffe, wie Dibromdicyanobutan (2-Brom-2-brommethylglutarodinitril), Phenoxyethanol, 3-lod-2-propinylbutylcarbamat, 2-Brom-2-nitro-propan-1,3-diol, Imidazolidinylharnstoff, 5 -Chlor-2-methyl-4-isothiazolin-3-on, 2 -Chloracetamid, Benzalkoniumchlorid, Benzylalkohol.

Es ist dabei erfindungsgemäß besonders bevorzugt, wenn als Konservierungsstoffe Benzoesäure und/oder Salicylsäure und/oder deren Derivate oder Salze eingesetzt werden.

Erfindungsgemäß vorteilhaft sind ein oder mehrere Konservierungsstoffe in einer Konzentration von 2 Gewichts-% oder kleiner 2 Gewichts-%, bevorzugt 1,5 Gewichts-% oder kleiner 1,5 Gewichts-% und besonders bevorzugt 1 Gewichts-% oder kleiner 1 Gewichts-%, jeweils bezogen auf das Gesamtgewicht der Zubereitung A oder B in einer der beiden Teilzubereitungen oder in beiden Teilzubereitungen enthalten.

Die erfindungsgemäßen Zubereitungen enthalten vorteilhafter Weise einen oder mehrere Konditionierer. Erfindungsgemäß bevorzugte Konditionierer sind beispielsweise alle Verbindungen, welche im International Cosmetic Ingredient Dictionary and Handbook (Volume 4, Herausgeber: R. C. Pepe, J.A. Wenninger, G. N. McEwen, The Cosmetic, Toiletry, and Fragrance Association, 9. Auflage, 2002) unter Section 4 unter den Stichworten Hair Conditioning Agents, Humectants, Skin-Conditioning Agents, Skin-Conditioning Agents-Emollient, Skin-Conditioning Agents-Humactant, Skin-Conditioning Agents-Miscellaneous, Skin-Conditioning Agents-Occlusive und Skin Protectans aufgeführt sind sowie alle in der EP 0934956 (S.11-13) unter water soluble conditioning agent und oil soluble conditioning agent aufgeführten Verbindungen. Ein Teil dieser Verbindungen wird unter den Bestandteilen der wässrigen Phase und der Ölphase namentlich aufgeführt. Weitere erfindungsgemäß vorteilhafte Konditionierer stellen beispielsweise die nach der internationalen Nomenklatur für kosmetische Inhaltsstoffe (INCI) als Polyquaternium benannten Verbindungen dar (insbesondere Polyquaternium-1 bis Polyquaternium-56).

Eine besonders bevorzugte Ausführungsform der vorliegenden Erfindung stellen Textilien dar, die mit einer erfindungsgemäßen Emulsion getränkt sind. Dabei ist es erfindungsgemäß bevorzugt, wenn das Textil in Form eines ungewebten Vlieses, insbesondere in Form eines Tuches oder "Pads", vorliegt.

Erfindungsgemäß bevorzugt werden Textilien eingesetzt, welche aus Vlies bestehen, insbesondere aus wasserstrahlverfestigten und/oder wasserstrahlgeprägten Vlies.

Die erfindungsgemäßen Textilien können glatt oder auch oberflächenstrukturiert (beispielsweise genoppt oder gelocht) sein. Erfindungsgemäß bevorzugt sind oberflächenstrukturierte Textilien.

Derartige Textilien können Makroprägungen jeden gewünschten Musters aufweisen. Die zu treffende Auswahl richtet sich zum einen nach der aufzubringenden Tränkung und zum anderen nach dem Einsatzfeld, auf dem das spätere Textil Verwendung finden soll.

Werden geprägte Vliese verwendet, so erleichtern große Kavitäten an der Vliesoberfläche und im Vlies die Aufnahme von Schmutz und Verunreinigungen, wenn mit dem getränkten Tuch über die Haut gefahren wird. Die Reinigungswirkung kann gegenüber ungeprägten Tüchern um ein Vielfaches gesteigert werden.

Es hat sich als vorteilhaft herausgestellt für das Tuch, wenn dieses ein Gewicht von 35 bis 120 g/m², vorzugsweise von 40 bis 60 g/m², hat (gemessen bei 20°C ± 2°C und bei einer Feuchtigkeit der Raumluft von 65 % ± 5 % für 24 Stunden).

Die durchschnittliche Dicke des Vlieses beträgt vorzugsweise 0,4 mm bis 2 mm, insbesondere 0,6 mm bis 1,2 mm (gemessen nach der Methode ERT 30.5-99).

Als Ausgangsmaterialien für den Vliesstoff des Textils können neben den erfindungsgemäßen Faserstoffen generell alle organischen und anorganischen Faserstoffe auf natürlicher und synthetischer Basis verwendet werden. Beispielhaft seien Zellulose, Jute, Hanf, Sisal, Seide, Wolle, Polypropylen, Polyethylenterephthalat (PET), Aramid, Nylon, Polyvinylderivate, Polyurethane, Polylactid, Polyhydroxyalkanoat, Celluloseester und/oder Polyethylen sowie auch mineralische Fasern wie Glasfasern oder Kohlenstoffasern angeführt. Die vorliegende Erfindung ist aber nicht auf die genannten Materialien beschränkt, sondern es können eine Vielzahl weiterer Fasern zur Vliesbildung eingesetzt werden. Es ist insbesondere vorteilhaft im Sinne der vorliegenden Erfindung, wenn die eingesetzten Fasern nicht wasserlöslich sind.

Darüber hinaus können die Fasern auch eingefärbt sein, um die optische Attraktivität des Vlieses betonen und/oder erhöhen zu können. Die Fasern können zusätzlich UV-Stabilsatoren und/oder Konservierungsmittel enthalten.

Die zur Bildung des Tuches eingesetzten Fasern weisen vorzugsweise eine Wasseraufnahmerate von mehr als >20 mm/[10 min] (gemessen mit dem EDANA Test 10.2-96), auf.

Ferner weisen die zur Bildung des Tuches eingesetzten Fasern vorzugsweise ein Wasseraufnahmevermögen von mehr als >9 g/g (gemessen mit dem EDANA Test 10.2-96), auf.

Vorteilhafte Tücher im Sinne der vorliegenden Erfindung haben eine Reißkraft von insbesondere (gemessen nach der Methode ERT 20.2-89)

| | | [N/50mm] |
|---|---|---|
| im trockenen Zustand | Maschinenrichtung | >70, vorzugsweise >80 |
| | Querrichtung | >28, vorzugsweise >30 |
| im getränkten Zustand | Maschinenrichtung | >50, vorzugsweise >60 |
| | Querrichtung | >24, vorzugsweise >30 |

Die Dehnfähigkeit vorteilhafter Tuches beträgt vorzugsweise (gemessen nach der Methode ERT 20.2-89)

| | | |
|---|---|---|
| im trockenen Zustand | Maschinenrichtung | 45 +/- 15% |
| | Querrichtung | 110 +/- 20% |
| im getränkten Zustand | Maschinenrichtung | 45 +/- 15% |
| | Querrichtung | 90 +/- 20% |

Erfindungsgemäß vorteilhaft beträgt der Tränkungsgrad des Reinigungsartikels, d.h. das Gewichtsverhältnis von kosmetischer Zubereitung (=flüssiger Tränkung) und Textil, von 2,1 bis 4,0 , bevorzugt von 2,4 bis 3,7 und besonders bevorzugt von 2,7 bis 3,4.

In einer vorteilhaften Ausführungsform des Vlieses bestehen die Fasern aus einer Mischung aus ungefähr 70 % Viskose und 30 % PET.

Erfindungsgemäß bevorzugt ist ein Textil, welches aus einer Mischung von Viskosefasern, Polyesterfasern und/oder Baumwollfasern gebildet wird.

Vorteilhaft im Sinne der vorliegenden Erfindung ist es insbesondere, wenn der Textilstoff des erfindungsgemäßen Textils gebildet wird aus
1-30 Gewichts-% Baumwollfasern,
9-80 Gewichts-% Viskosefasern und
19-90 Gewichts-% Polyester,
jeweils bezogen auf das Gesamtgewicht des Textils.

Ganz besonders bevorzugt weist das erfindungsgemäße Textil an der Oberfläche einen Baumwollanteil bis 30 Gewichts-% und im Inneren einen Baumwollanteil bis 10 Gewichts-%, jeweils bezogen auf das Gesamtgewicht des Textils, auf.

Erfindungsgemäß vorteilhaft beträgt der Ttränkungsgrad zwischen 2 und 4 und erfindungsgemäß bevorzugt zwischen 2,8 und 3,4. Dabei bedeutet ein Tränkungsgrad von 2,8 das 1g Textil 2,8 g Tränkungsmedium enthalten.

Erfindungsgemäß vorteilhaft ist aber auch der Einsatz der erfindungsgemäßen Emulsion als sprühbarer Emulsion. In einem solchen Falle kann die erfindungsgemäße Emulsion mit Hilfe eines Druckgases aus einer Aerosoldose oder mit Hilfe einer Pumpe aus einem Pumpspray-Applikator heraus angewendet werden.

Erfindungsgemäß ist die Verwendung der erfindungsgemäßen Emulsion oder eines Textils oder Sprüh-Applikator zur Reinigung der Haut, insbesondere der Gesichtshaut. Die folgenden Beispiele, sollen die erfindungsgemäßen Zusammensetzungen erläutern, ohne dass aber beabsichtigt ist, die Erfindung auf diese Beispiele zu beschränken. Die Zahlenwerte in den Beispielen bedeuten Gewichtsprozente, bezogen auf das Gesamtgewicht der jeweiligen Zubereitungen.

### Beispiele

| | **(A) m[%]** | **(B) m[%]** | **(C) m[%]** |
|---|---|---|---|
| Acrylates/C10-30 Alkyl Acrylate Crosspolymer | 0,1 | 0,1 | |
| Carbomer | | | 0,15 |
| Cetearylisononanoat | 4,0 | | 2,0 |
| Dimethicon | | 5,0 | 3,0 |
| Fragrance | 0,2 | 0,4 | 0,3 |
| Glycerin | 4,0 | 7,0 | 3,0 |
| Glycerylstearatcitrat | 1,0 | | |
| Isohexadecan | 3,0 | 3,3 | 4,5 |
| Isopropylisostearat | 2,0 | | 2,0 |
| Isopropylpalmitat | | 2,5 | |
| Methylparaben | 0,25 | 0,2 | 0,30 |
| Phenoxyethanol | 0,5 | 0,5 | 0,2 |
| Polyglyceryl-3 Methylglucose Distearat | | 0,4 | |
| Propylparaben | 0,15 | 0,15 | 0,15 |
| Triceteareth-4 Phosphat | | | 1,0 |
| Natriumhydroxid | qs | qs | Qs |
| Wasser | ad 100 | ad 100 | ad 100 |

Verwendung der Medien zur Tuchtränkung, Viskose/Polyester 10:90 - 90:10, 40g/m²-80g/m² , Tränkungsgrad 3,2.

## Patentansprüche

1. Kosmetische und/oder dermatologische Öl-in-Wasser- (O/W-) Emulsion mit einer Viskosität von 10 bis 1500 mPas, enthaltend
a) einen oder mehrere Emulgatoren gewählt aus der Gruppe Glycerylstearatcitrat, Polyglycerinmethylglucosedistearat, Triceteareth-4-phosphat, b) ein Homo- oder Copolymer der Acrylsäure und/oder ihrer Derivate als polymeren Stabilisator,
wobei die Gesamtkonzentration der Summe aus Emulgator a) und polymerem Stabilisator b) zwischen 0,01 und 1,5 Gewichts-%, bezogen auf das Gesamtgewicht der Emulsion beträgt und die Emulsion frei ist von Fettalkoholen mit einer Kettenlänge von 12 bis 20 Kohlenstoffatomen.

2. Emulsion nach Anspruch 1, **dadurch gekennzeichnet, dass** als Emulgator a) Glycerylstearatcitrat eingesetzt wird.

3. Emulsion nach Anspruch 1, **dadurch gekennzeichnet, dass** als Emulgator a) Polyglycerinmethylglucosedistearat eingesetzt wird.

4. Emulsion nach Anspruch 1, **dadurch gekennzeichnet, dass** als Emulgator a) Triceteareth-4-phosphat eingesetzt wird.

5. Emulsion nach einem der Ansprüche 1 bis 4, **dadurch gekennzeichnet, dass** als polymerer Stabilisator Polyacrylate und/oder Acrylat/C₁₀-C₃₀Alkylacrylat-Copolymer eingesetzt wird.

6. Emulsion nach einem der Ansprüche 1 bis 5, **dadurch gekennzeichnet, dass** die Konzentration an polymeren Stabilisator 0,001 bis 0,1 Gewichts-%, bezogen auf das Gesamtgewicht der Zubereitung beträgt.

7. Textil getränkt mit einer Emulsion nach einem der Ansprüche 1 bis 6.

8. Textil nach Anspruch 7, **dadurch gekennzeichnet, dass** das Textil in Form eines ungewebten Vlieses, insbesondere in Form eines Tuches, vorliegt.

9. Textil nach einem der Ansprüche 7 oder 8, **dadurch gekennzeichnet, dass** das Textil aus einer Mischung von Viskosefasern, Polyesterfasern und/oder Baumwollfasern gebildet wird.

10. Verwendung einer Zubereitung nach einem der Ansprüche 1 bis 6 oder eines Textils nach einem der Ansprüche 7 bis 9 zur Reinigung der Haut, insbesondere der Gesichtshaut.

## Claims

1. Cosmetic and/or dermatological oil-in-water (O/W) emulsion with a viscosity of from 10 to 1500 mPas, comprising
a) one or more emulsifiers selected from the group glyceryl stearate citrate, polyglycerol methylglucose distearate, triceteareth-4 phosphate, b) a homopolymer or copolymer of acrylic acid and/or its derivatives as polymeric stabilizer,
where the total concentration of the sum of emulsifier a) and polymeric stabilizer b) is between 0.01 and 1.5% by weight, based on the total weight of the emulsion, and the emulsion is free from fatty alcohols with a chain length of from 12 to 20 carbon atoms.

2. Emulsion according to Claim 1, **characterized in that** glyceryl stearate citrate is used as emulsifier a).

3. Emulsion according to Claim 1, **characterized in that** polyglycerol methylglucose distearate is used as emulsifier a).

4. Emulsion according to Claim 1, **characterized in that** triceteareth-4 phosphate is used as emulsifier a).

5. Emulsion according to one of Claims 1 to 4, **characterized in that** polyacrylates and/or acrylate/C₁₀-C₃₀-alkyl acrylate copolymer is used as polymeric stabilizer.

6. Emulsion according to one of Claims 1 to 5, **characterized in that** the concentration of polymeric stabilizer is 0.001 to 0.1% by weight, based on the total weight of the preparation.

7. Textile impregnated with an emulsion according to one of Claims 1 to 6.

8. Textile according to Claim 7, **characterized in that** the textile is present in the form of a nonwoven fabric, in particular in the form of a wipe.

9. Textile according to one of Claims 7 and 8, **characterized in that** the textile is formed from a mixture of viscose fibres, polyester fibres and/or cotton fibres.

10. Use of a preparation according to one of Claims 1 to 6 or of a textile according to one of Claims 7 to 9 for cleaning the skin, in particular the facial skin.

## Revendications

1. Émulsion huile-dans-eau (HIE) cosmétique et/ou dermatologique ayant une viscosité de 10 à 1 500 mPa.s, contenant
a) un ou plusieurs émulsifiants choisis dans le groupe constitué par le glycéryl stéarate citrate, le polyglycéryl-méthylglucose distéarate, le tricétéareth-4-phosphate,
b) un homo- ou copolymère de l'acide acrylique et/ou ses dérivés en tant que polymère stabilisant,
la concentration totale de la somme de l'émulsifiant a) et du stabilisant polymère b) étant comprise entre 0,01 et 1,5 % en poids, par rapport au poids total de l'émulsion et l'émulsion étant exempte d'alcools gras ayant une longueur de chaîne de 12 à 20 atomes de carbone.

2. Émulsion selon la revendication 1, **caractérisée en ce qu'**on utilise comme émulsifiant a) le glycéryl stéarate citrate.

3. Émulsion selon la revendication 1, **caractérisée en ce qu'**on utilise comme émulsifiant a) le polyglycéryl-méthylglucose distéarate.

4. Émulsion selon la revendication 1, **caractérisée en ce qu'**on utilise comme émulsifiant a) le tricétéareth-4-phosphate.

5. Émulsion selon l'une quelconque des revendications 1 à 4, **caractérisée en ce qu'**on utilise comme stabilisant polymère des polyacrylates et/ou un copolymère acrylate/acrylate d'alkyle en C₁₀-C₃₀.

6. Émulsion selon l'une quelconque des revendications 1 à 5, **caractérisée en ce que** la concentration du stabilisant polymère vaut de 0,001 à 0,1 % en poids, par rapport au poids total de la préparation.

7. Textile imprégné avec une émulsion selon l'une quelconque des revendications 1 à 6.

8. Textile selon la revendication 7, **caractérisé en ce que** le textile se trouve sous forme d'un voile non tissé, en particulier sous forme d'une lingette.

9. Textile selon l'une quelconque des revendications 7 ou 8, **caractérisé en ce que** le textile est constitué d'un mélange de fibres de viscose, de fibres de polyester et/ou de fibres de coton.

10. Utilisation d'une préparation selon l'une quelconque des revendications 1 à 6 ou d'un textile selon l'une quelconque des revendications 7 à 9, pour le nettoyage de la peau, en particulier de la peau du visage.
